# EUROPEAN PATENT APPLICATION

(11) **EP 4 706 387 A1**
(43) Date of publication of application: **11.03.2026**
(21) Application number: 24803491.0
(22) Date of filing: 08.05.2024
(51) Int. Cl.: A01K 67/027, A01K 35/00, C12N 15/12

(54) **METHOD FOR SEXING BIRDS, BIRDS, PRODUCTION METHOD, EGG POPULATION, AND SEXING DEVICE**

(30) Priority: 11.05.2023 JP 2023078539
(71) Applicant: Setsurotech Inc., Tokushima-shi, Tokushima, 770-0053 (JP)
(72) Inventor: CHEN, Yi-Chen, Tokushima-shi, Tokushima 770-8503 (JP); TAKEMOTO, Tatsuya, Tokushima-shi, Tokushima 770-8501 (JP); SHIMOKITA, Eisuke, Tokushima-shi, Tokushima 770-8501 (JP)
(74) Representative: J A Kemp LLP
(86) International application number: PCT/JP2024/017071
(87) International publication number: WO 2024/232382

(57) **Abstract**

A method that can be used for bird sexing is provided. A bird sexing method of the present disclosure includes a step of sexing a bird inside each egg of a bird egg population based on the color of the eyes of the bird. When the bird inside the egg is a male bird, the male bird is heterozygous for an eye pigment-expression gene on the Z chromosome and has a loss-of-function pigment-expression gene and a wild-type eye pigment-expression gene. When the bird inside the egg is a female bird, the female bird has a loss-of-function eye pigment-expression gene on the Z chromosome.

## Description

### TECHNICAL FIELD

The present disclosure relates to a bird sexing method, a bird, a production method, an egg population, and a sexing apparatus.

### BACKGROUND ART

After hatching, chickens are sexed for commercial purposes. After the chickens have been sexed, female chickens, which will lay eggs, are delivered to egg-collection poultry farms, whereas male chickens, which do not lay eggs, are, for example, killed. Accordingly, killing of male chickens has become a worldwide problem (Non-Patent Literature 1).

Also, in Europe, the killing of chickens is required to be performed up to 7 days after egg-laying, that is to say, before the nervous system develops. Although an attempt has been made to develop a technology that enables sexing up to 7 days after egg-laying, a technology in which no biomarkers are used or no invasion is involved has not been established yet.

### Citation List

### Non-Patent Literature

Non-Patent Literature 1: General Secretariat of the Council, "EU-wide end to the systematic killing of male chicks - Information from the French and German delegations on behalf of the Austrian, Belgian, Cyprus, Finnish, French, German, Irish, Luxembourg and Portuguese delegations", [online], 12 October 2022, the Council ("Agriculture and Fisheries"), [searched on May 11, 2023], on the Internet <URL: https://data.consilium.europa.eu/doc/document/ST-13317-2022-INIT/x/pdf>

### SUMMARY OF INVENTION

### Technical Problem

Therefore, it is an object of the present disclosure to provide a method with which a bird can be sexed, an egg population to be subjected to sexing, a bird to be used to produce the egg population or a method for producing the bird, or an apparatus to be used to sex a bird.

### Solution to Problem

In order to achieve the object, a bird sexing method of the present disclosure includes a step of sexing a bird inside each egg of a bird egg population based on a color of eyes of the bird,
wherein when the bird inside the egg is a male bird, the male bird is heterozygous for an eye pigment-expression gene on a Z chromosome and has a loss-of-function pigment-expression gene and a wild-type eye pigment-expression gene, and
when the bird inside the egg is a female bird, the female bird has a loss-of-function eye pigment-expression gene on a Z chromosome.

A bird of the present disclosure has a loss-of-function eye pigment-expression gene on a Z chromosome.

A bird production method (also referred to as a "first production method" hereinafter) of the present disclosure includes a step of mating a male bird with a female bird,
wherein the male bird is the bird of the present disclosure, and
the female bird has a wild-type eye pigment-expression gene on a Z chromosome.

A bird production method (also referred to as a "second production method" hereinafter) of the present disclosure includes a step of mating a male bird with a female bird,
wherein the male bird and/or the female bird is the bird of the present disclosure.

A bird production method (also referred to as a "third production method" hereinafter) of the present disclosure includes:
a step of sexing a bird inside each egg of a bird egg population based on the color of the eyes of the bird; and
a step of obtaining a progeny using the bird that has been sexed,
wherein the sexing is conducted using the bird sexing method of the present disclosure.

A bird egg population of the present disclosure is a bird egg population,
wherein when a bird inside the egg is a male bird, the male bird is heterozygous for the eye pigment-expression gene on the Z chromosome and has the loss-of-function pigment-expression gene and the wild-type eye pigment-expression gene, and
when a bird inside the egg is a female bird, the female bird has the loss-of-function eye pigment-expression gene on the Z chromosome.

A sexing apparatus of the present disclosure is a sexing apparatus including:
a light source for irradiating a sexing target egg selected from a bird egg population with light;
an imaging unit for imaging the sexing target egg in a state in which a content thereof is visible due to irradiation with light from the light source and outputting image data containing an eye of a bird inside the sexing target egg; and
a sexing unit for sexing the bird inside the sexing target egg based on the image data.

### Advantageous Effects of Invention

With the present disclosure, it is possible to provide a method with which a bird can be sexed, an egg population to be subjected to sexing, a bird to be used to produce the egg population or a method for producing the bird, or an apparatus to be used to sex a bird.

### Brief Description of Drawings

[FIG. 1] FIG. 1 is a diagram showing the genotype of the target region of the SLC45A2 gene of a clone obtained in Example 1.
[FIG. 2] FIG. 2 shows photographs illustrating the color of the eye of a chicken 7 days after the start of egg incubation in Example 1.
[FIG. 3] FIG. 3 shows photographs illustrating the color of the eye of a chicken 10 days after the start of egg incubation in Example 1.
[FIG. 4] FIG. 4 is a block diagram illustrating a sexing apparatus of an embodiment.
[FIG. 5] FIG. 5 is a schematic diagram illustrating the sexing apparatus of the embodiment.
[FIG. 6] FIG. 6 is a diagram illustrating a state of the sexing apparatus of the embodiment in which an egg to be sexed is moved to a darkroom.
[FIG. 7] FIG. 7 is a flowchart illustrating sexing processing of the embodiment.
[FIG. 8] FIG. 8 is a schematic diagram illustrating, with a broken line, an eyeball region extracted from image data through eyeball detection processing in the sexing apparatus of the embodiment.

### DESCRIPTION OF EMBODIMENTS

### <Definitions>

The term "bird" as used herein means an animal that is classified into the class Aves of the phylum Vertebrata.

The term "loss of function" as used herein means, for example, a state in which the inherent function of a target gene decreases or is lost. The above-mentioned "loss of function" can also be referred to as, for example, "inactivation (or inactivated)".

The term "loss-of-function mutation" as used herein means a mutation that leads to a (significant) reduction in the inherent function of a target gene and/or complete loss of function. The above-mentioned "mutation that leads to complete loss of function" can also be referred to as, for example, "null mutation" or "amorph".

The term "bird" as used herein means an individual of birds.

The term "a portion of a bird" as used herein means a part or portion of an individual bird.

The term "eye pigment-expression gene" means a gene that controls the expression of an eye pigment. The above-mentioned eye pigment-expression gene may also control eye pigment expression in tissues other than the eyes.

The term "sex chromosome" as used herein means a chromosome that varies in shape or number between a female and a male of an organism with sex differentiation. The sex chromosome of the bird means the Z chromosome and/or the W chromosome.

The term "population" as used herein means a group consisting of two or more constituents.

Information on the sequences of proteins described in this specification or the sequences of nucleic acids (e.g., DNAs or RNAs) encoding the proteins is available from Protein Data Bank, UniProt, Ensembl, GenBank, or the like. In addition, the nucleic acid sequences of RNAs can be obtained from the nucleic acid sequences of the corresponding DNAs using sequence conversion software as appropriate.

Although the following describes examples of the present disclosure, the present disclosure is not limited to the following examples and the like, and can be implemented with any modifications. Also, each description in the present disclosure can be applied to other descriptions unless otherwise stated. Note that the expression "to" as used herein means that the numerical or physical values before and after "to" are included. In addition, the expression "A and/or B" as used herein encompasses "only A", "only B", and "both A and B".

### <Bird>

In a certain aspect, the present disclosure provides a bird in which the function of an eye pigment-expression gene on the Z chromosome is lost. In the bird of the present disclosure, the function of the eye pigment-expression gene is lost.

As a result of extensive research, the inventors of the present invention came up with an idea that the eye pigment-expression gene on the sex chromosome can be used to change the color of the eyes according to sex of a bird. The inventors of the present invention found that the eye pigment-expression gene on the Z chromosome, which is the sex chromosome of a bird, can be used to change the color of the eyes according to sex of a bird and sexing can be performed based on the color of the eyes, and thus established the present disclosure. Specifically, it was found that, when a progeny bird is obtained by mating a male bird that is homozygous for the eye pigment-expression gene and in which the function of the eye pigment-expression gene is lost with a female bird that has the wild-type eye pigment-expression gene on the Z chromosome, the progeny bird can be sexed based on the color of the eyes of the progeny bird. In general, vent sexing or feather sexing has been performed as the bird sexing, but these methods can be performed only after hatching. In contrast, with the present disclosure, it is possible to sex a bird even before hatching, and it is expected that a bird can be sexed before the nervous system of the bird develops, that is to say, for example, within 7 days after the start of egg incubation using the day on which the egg is laid as a reference date (0 days after the start of egg incubation).

Examples of the bird include birds belonging to the families Phasianidae, Anatidae, Struthionidae, Columbidae, Numididae, Casuariidae, and the like. Examples of the birds belonging to the family Phasianidae include a chicken (Gallus gallus domesticus), a quail (Coturnix japonica), a turkey (Meleagris gallopavo), a pheasant (Phasianus versicolor), and the like. Examples of the birds belonging to the family Anatidae include a domestic duck (Anas platyrhynchos var. domesticus), a duck (Anas), a goose (Anser anser domesticus), and the like. Examples of the birds belonging to the family Struthionidae include an ostrich (Struthio camelus) and the like. Examples of the birds belonging to the family Columbidae include a dove (Columbidae) and the like. Examples of the birds belonging to the family Numididae include a guinea fowl (Numida meleagris) and the like. Examples of the birds belonging to the family Casuariidae include an emu (Dromaius novaehollandiae) and the like. Examples of the breeds of the chicken include White Leghorn, Brown Leghorn, Barred Rock, Sussex, New Hampshire, Rhode Island, Australorp, Minorca, Amrox, California Gray, Italian Partridge colored, Korean Oge, and the like.

In the present disclosure, the bird may be a true breed or a crossbreed. The crossbreed may be a crossbreed obtained through intergeneric crossing or interspecific crossing.

In the present disclosure, it is preferable that the bird is, for example, a livestock bird or a domestic fowl.

In the present disclosure, the eye pigment-expression gene is not particularly limited as long as it is a gene capable of controlling the expression of an eye pigment. The expression control may be induction, promotion, or enhancement of the expression or function of a target gene or a protein encoded by the gene; suppression, decrease, or cessation of the expression or function of a gene of interest, or the target gene or a protein encoded by the gene; or the like.

In the present disclosure, the eye pigment-expression gene on the Z chromosome (also referred to as the "eye pigment-expression gene" hereinafter) may be, for example, the SLC45A2 (solute carrier family 45 member 2) gene, or the like. The SLC45A2 gene has a function of enabling, for example, induction, promotion, or enhancement of the eye pigment expression. The eye pigment is, for example, eumelanin and/or pheomelanin. By using the SLC45A2 gene as the eye pigment-expression gene on the Z chromosome, when the bird is male and is homozygous for the loss-of-function eye pigment-expression gene (loss-of-function gene), the color of the eyes of the bird can be made transparent, for example, in the egg. Also, when the bird is female and has the loss-of-function eye pigment-expression gene, the color of the eyes of the bird can be made transparent, for example, in the egg. Meanwhile, when these birds grow mature, for example, outside the eggs, the color of the eyes thereof is substantially the same as that of birds having the wild-type eye pigment-expression gene.

The SLC45A2 is commonly known as a proton-associated glucose and sucrose transporter. It is presumed that the SLC45A2 gene maintains the osmotic pressure in a melanosome through glucose-sucrose transport and maintains the pH in the melanosome, and thus promotes the conversion to active tyrosinase through coupling of inactive tyrosinase and a copper ion. In the present disclosure, the mRNA from the chicken SLC45A2 gene is, for example, a polynucleotide having a base sequence registered in GenBank with the accession number: NM_001083364.3, and the protein encoded by the chicken SLC45A2 gene is, for example, a polypeptide having the amino acid sequence registered in GenBank with the accession number: NP_001076833.3.

A specific example of the chicken SLC45A2 gene (wild-type SLC45A2 gene) is a polynucleotide (S) below or a genome region encoding the polynucleotide (S). Note that the base sequence of SEQ ID NO: 1 below includes a stop codon.

(S) Any of polynucleotides (S1) to (S7) below:
(S1) a polynucleotide having a base sequence of SEQ ID NO: 1;
(S2) a polynucleotide that encodes a protein having an eye pigment expression function and that has a base sequence consisting of the base sequence of (S1) above with deletion, substitution, insertion, and/or addition of one or several bases;
(S3) a polynucleotide that encodes a protein having an eye pigment expression function and that has a base sequence having 80% or more identity to the base sequence of (S1) above;
(S4) a polynucleotide that encodes a protein having an eye pigment expression function and that has a base sequence complementary to a polynucleotide capable of hybridizing to the polynucleotide having the base sequence of (S1) above under a stringent condition;
(S5) a polynucleotide encoding a protein having an amino acid sequence of SEQ ID NO: 2;
(S6) a polynucleotide encoding a protein that has an eye pigment expression function and that has an amino acid sequence consisting of the amino acid sequence of SEQ ID NO: 2 with deletion, substitution, insertion, and/or addition of one or several amino acids; and
(S7) a polynucleotide encoding a protein that has an eye pigment expression function and that has an amino acid sequence having 80% or more identity to the amino acid sequence of SEQ ID NO: 2.
   Base Sequence of Chicken SLC45A2 Gene (SEQ ID NO: 1)

5'-
ATGACCCTAACGGAACAGCACTTCCAGGAGTCTCCTCCACCCCCTTCTGAGGTGGCC
AAGGCAGACATGGACAGCACAGGAGGGAAGGAGAAGGCTGCGCAGCCCTCCACGG
CAAGGACTGGAGCGCTGGTGCGCAAGCAGCAAGCAACGGGAAGGCTGATCATGCAC
AGCATGGCGATGTTCGGGAGGGAGTTCTGCTATGCTGTGGAGGCTGCCTTTGTCACG
CCAGTGCTGCTCAGTGTAGGGCTGCCCAAGAGCCTGTACAGCCTGGTGTGGCTCATC
AGCCCCATCCTGGGCTTCGTGCTGCAGCCCGTGGTAGGTTCTGCCAGTGATCACTGC
GCCTGTAGCTGGGGCAGGAGGCGACCTTACATTCTGGGTCTGGGCATCATAATGCTGG
TAGGAATGGCTTTGTACCTCAATGGGGACGAGATGATCTCAGCTTTCATCGGTGAGAG
AGAGAAGCAACGGACATGGGCAATAGTCATTACCATGCTGGGAGTAGTGCTTTTTGAT
TTTGCAGCTGACTTTATTGATGGCCCCATCAAAGCATATTTATTTGACGTCTGCTCTCA
TGAAGATAAAGAGAAGGGTCTGCATTATCATGCCCTGTTTACAGGCTTAGGAGGAGC
ACTGGGCTACCTGACAGGTGCTGTGGACTGGGGTGAAACTGTACTAGGATATTCCTTG
ACGTCAGAATTCCAGGTGATTTTCCTCTTGTCAGCCTTGGTTTTCCTCATCTGCCTTAT
TGTACATCTACGCAGTATTCCTGAAGTCCCACTCAGATATGGCAACAAAGAGACAAA
GCTCTTGTTGGAAGTGACCGAGCCCTATAAGTACAGATCCATAGAGGAAATCAAGAAT
GGATACTCGTCATGTACTGACTTAAATGCTACGAGTAAGACAAAGAAAGGTACTGATG
CATCGTGTTCAGAGGCTCAAAGGCGGATGACACTTAAGTCACTCTTGAAGACTCTTTT
AAGCATGCCATCCCATTATCGCTGTCTGTGTGTGAGCCACCTCTTTGGATGGATGGCTT
TCATGTCCAACATGCTCTTCTTCACGGATTTCATGGGGCAGGTTGTGTACCAGGGCAG
TCCTTATGCTTCTCATAACTCCACACTATACCATACCTACAGAAGAGGAGTGGAGGTT
GGATGCTGGGGGCTGTGCATCAATGCGATTGCATCCTCAGCCTATTCTTACCTGCAGA
AAGTCCTTCTGCCATACATAGGATTAAAGGGACTTTATTTCATTGGATACCTACTTTTT
GGATTAGGCACTGGGTTGATTGGCTTGTTTCCCAATGTCTATTCCACCTTGGTTCTATG
TTCGCTTTTTGGAGTCATGTCCAGCACTCTGTACACAGTGCCTTTCCAACTCATTGCA
GAGTATCACAAAGAAGAAGAGGACCTGAATCTGCAGCAGAAGGAACAAGGCACAG
AGCATGGGAGAGGAAAGGGCATTGACTGTGCTGCTCTCACCTGCATGGTCCAGCTGG
CACAGATCATTCTCGGTGTGGGCCTAGGGCTCTTGGTCAGTGTTGCTGGCAGTGCGGT
CACTGTGATTTCAGCATCAATGGTGGCACTGATTGGCTGCTGCTTTGTTGCTTTCTGTG
TTCGATATGTGGGGTAA-3'

In (S1) above, the base sequence of SEQ ID NO: 1 is the base sequence encoding the amino acid sequence (SEQ ID NO: 2) of (S5) above. The base sequence of SEQ ID NO: 1 can be obtained from, for example, a chicken (Gallus gallus domesticus).

In (S2) above, the "one or several" bases may be any number of bases as long as, for example, the protein encoded by the polynucleotide of (S2) above has the eye pigment expression function. The above-mentioned eye pigment expression function means a function of enabling, for example, induction, promotion, or enhancement of the eye pigment expression (the same applies hereinafter). The "one or several" bases in (S2) above refer to, for example, 1 to 326 bases, 1 to 244 bases, 1 to 163 bases, 1 to 81 bases, 1 to 65 bases, 1 to 48 bases, 1 to 32 bases, 1 to 16 bases, 1 to 8 bases, 1 to 5 bases, 1 to 3 bases, 1 or 2 bases, or 1 base, in the base sequence of (S1) above. In the present disclosure, the numerical range of the number of bases, amino acids, or the like discloses, for example, all positive integers belonging to that range. Specifically, for example, "1 to 5" means disclosure of all of "1, 2, 3, 4, and 5" (the same applies hereinafter).

In (S3) above, the "identity" may be any degree of identity as long as, for example, the protein encoded by the polynucleotide of (S3) above has the eye pigment expression function. In (S3) above, the identity to the base sequence of (S1) above is, for example, 80% or more, 85% or more, 90% or more, 95% or more, 96% or more, 97% or more, 98% or more, or 99% or more. The "identity" can be determined through alignment of two base sequences or two amino acid sequences (the same applies hereinafter). The above-mentioned alignment can be calculated using, for example, BLAST, FASTA, or the like with default parameters.

The "polynucleotide capable of hybridizing" in (S4) above is not limited as long as, for example, the protein encoded by the polynucleotide of (S4) above has the eye pigment expression function. In (S4) above, the "polynucleotide capable of hybridizing" is, for example, a polynucleotide that is fully or partially complementary to the polynucleotide of (S1) above. The hybridization can be detected, for example, through various hybridization assays. There is no particular limitation on the above-mentioned hybridization assays, and methods described in, for example, Sambrook et al. ed. "Molecular Cloning: A Laboratory Manual 2nd Ed." (Cold Spring Harbor Laboratory Press (1989)), etc., can also be employed.

In (S4) above, the "stringent condition" may be, for example, any of a low-stringent condition, a medium-stringent condition, and a high-stringent condition. The "low-stringent condition" is, for example, a condition that 5×SSC, 5×Denhardt's solution, 0.5% SDS, and 50% formamide are used, and the temperature is set to 32°C. The "medium-stringent condition" is, for example, a condition that 5×SSC, 5×Denhardt's solution, 0.5% SDS, and 50% formamide are used, and the temperature is set to 42°C. The "high-stringent condition" is, for example, a condition that 5×SSC, 5×Denhardt's solution, 0.5% SDS, and 50% formamide are used, and the temperature is set to 50°C. A person skilled in the art can determine the degree of stringency by, for example, selecting conditions such as the temperature, the salt concentration, the concentration and length of a probe, the ionic strength, and the time as appropriate. The conditions described in, for example, Sambrook et al. ed. "Molecular Cloning: A Laboratory Manual 2nd Ed." (Cold Spring Harbor Laboratory Press (1989)), etc., described above can also be employed as the "stringent condition".

The polynucleotide of (S5) above may have any base sequence as long as, for example, the protein encoded by the polynucleotide of (S5) above has the eye pigment expression function. The base sequence of the polynucleotide of (S5) above can be designed through, for example, conversion to corresponding codons based on the amino acid sequence of SEQ ID NO: 2.
Amino Acid Sequence of Chicken SLC45A2 Protein (SEQ ID NO: 2)

MTLTEQHFQESPPPPSEVAKADMDSTGGKEKAAQPSTARTGALVRKQQATGRLIMHSMA
MFGREFCYAVEAAFVTPVLLSVGLPKSLYSLVWLISPILGFVLQPVVGSASDHCACSWGR
RRPYILGLGIIMLVGMALYLNGDEMISAFIGEREKQRTWAIVITMLGVVLFDFAADFIDGP
IKAYLFDVCSHEDKEKGLHYHALFTGLGGALGYLTGAVDWGETVLGYSLTSEFQVIFLL
SALVFLICLIVHLRSIPEVPLRYGNKETKLLLEVTEPYKYRSIEEIKNGYSSCTDLNATSKT
KKGTDASCSEAQRRMTLKSLLKTLLSMPSHYRCLCVSHLFGWMAFMSNMLFFTDFMG
QVVYQGSPYASHNSTLYHTYRRGVEVGCWGLCINAIASSAYSYLQKVLLPYIGLKGLYFI
GYLLFGLGTGLIGLFPNVYSTLVLCSLFGVMSSTLYTVPFQLIAEYHKEEEDLNLQQKEQ
GTEHGRGKGIDCAALTCMVQLAQIILGVGLGLLVSVAGSAVTVISASMVALIGCCFVAFC
VRYVG

In (S6) above, the "one or several" amino acids in the amino acid sequence may be any number of amino acids as long as, for example, the protein encoded by the polynucleotide of (S6) above has the eye pigment expression function. The "one or several" amino acids in (S6) above refer to, for example, 1 to 108 amino acids, 1 to 81 amino acids, 1 to 54 amino acids, 1 to 27 amino acids, 1 to 21 amino acids, 1 to 16 amino acids, 1 to 10 amino acids, 1 to 5 amino acids, 1 to 3 amino acids, 1 or 2 amino acids, or 1 amino acid, in the amino acid sequence of SEQ ID NO: 2.

In (S7) above, the "identity" of the amino acid sequence may be any degree of identity as long as, for example, the protein encoded by the polynucleotide of (S7) above has the eye pigment expression function. In (S7) above, the identity to the amino acid sequence of SEQ ID NO: 2 is, for example, 80% or more, 85% or more, 90% or more, 95% or more, 96% or more, 97% or more, 98% or more, or 99% or more.

In the present disclosure, the "eye pigment expression function" can be evaluated by, for example, directly or indirectly confirming the eye pigment of a target bird (test bird). The direct confirmation can be conducted by, for example, evaluating the eye pigment expression in the test bird visually or using an optical approach. In the indirect confirmation, evaluation can be conducted based on, for example, the expression of the eye pigment-expression gene or the eye pigment-expression protein in the biological sample from the test bird. The biological sample from the test bird is not particularly limited, and may be, for example, the individual test bird or a portion thereof, and the eye, the feather, and the like of the bird are preferable. When the biological sample from the test bird is a feather, it is preferable that the test bird belongs to a line in which a pigment is expressed in the feathers of a wild-type bird. For example, one type of biological sample or two or more types of biological samples may be used.

When the eye pigment expression function is evaluated visually or using an optical approach based on the eye pigment of the test bird, this evaluation can be conducted by, for example, evaluating the color of the eyes of the test bird after the eye development using, as a standard, the color of the eyes of a bird having the wild-type eye pigment-expression gene or a bird having the loss-of-function eye pigment-expression gene. Specifically, in accordance with Example 1 below, the color of the eyes of the test bird is visually observed after the eye development, or the color of the eyes of a bird inside an egg is determined by measuring the absorbance using an egg tester or the like. When the phenotype of the eye pigment of the test bird is the same as that of a male bird that is homozygous or heterozygous for the wild-type eye pigment-expression gene on the Z chromosome and/or a female bird that has the wild-type eye pigment-expression gene on the Z chromosome, it can be determined that, for example, the test bird has the eye pigment expression function. On the other hand, when the phenotype of the eye pigment of the test bird is the same as that of a male bird that is homozygous for the loss-of-function eye pigment-expression gene on the Z chromosome and/or a female bird that has the loss-of-function eye pigment-expression gene on the Z chromosome, it can be determined that, for example, the test bird does not have the eye pigment expression function. When the bird is a chicken, the eyes develop, for example, about 50 hours (about 3 days) after the start of egg incubation, and the eye pigment is expressed, for example, about 72 hours (about 3 days to about 4 days) after the start of egg incubation (Reference 1). Accordingly, when the bird is a chicken, the eye pigment expression function can be evaluated based on the eye pigment as long as the evaluation is conducted from 3 or 4 days, preferably 4 days, after the start of egg incubation onward. When the eye pigment expression function is evaluated visually or using an optical approach based on the eye pigment of the test bird, it is preferable to conduct the evaluation while irradiating the egg with light from the blunt end side (air chamber side) to the inside, because the color of the eyes can be more easily evaluated.

Reference 1: Hamburger V, Hamilton HL. A series of normal stages in the development of the chick embryo. 1951. Dev Dyn. 1992 Dec; 195(4): 231-72. doi: 10.1002/aja.1001950404. PMID: 1304821.

When the evaluation is conducted based on the expression of the eye pigment-expression gene, the expression of the eye pigment-expression gene can be detected by, for example, detecting the expression of the mRNA of the eye pigment-expression gene. The mRNA can be extracted from the biological sample from the test bird using an ordinary method. The expression of the mRNA of the eye pigment-expression gene can be detected through, for example, semi-quantitative PCR, quantitative PCR, northern blotting, digital PCR, RNA sequencing (RNAseq), or the like. Primers and/or probes used to detect the expression of the mRNA can be designed using, for example, common techniques in the art. When the expression level of the eye pigment-expression gene in the biological sample from the test bird is the same as (not significantly different from) the expression level of the eye pigment-expression gene in a male bird that is homozygous or heterozygous for the wild-type eye pigment-expression gene on the Z chromosome and/or a female bird that has the wild-type eye pigment-expression gene on the Z chromosome, and/or when the expression level of the eye pigment-expression gene in the biological sample from the test bird is (significantly) higher than the expression level of the eye pigment-expression gene in a male bird that is homozygous or heterozygous for the wild-type eye pigment-expression gene on the Z chromosome and/or a female bird that has the wild-type eye pigment-expression gene on the Z chromosome, and/or when the expression level of the eye pigment-expression gene in the biological sample from the test bird is (significantly) higher than the expression level of the eye pigment-expression gene in a male bird that is homozygous for the loss-of-function eye pigment-expression gene on the Z chromosome and/or a female bird that has the loss-of-function eye pigment-expression gene on the Z chromosome, it can be determined that, for example, the test bird has the eye pigment expression function. On the other hand, when the expression level of the eye pigment-expression gene in the biological sample from the test bird is (significantly) lower than the expression level of the eye pigment-expression gene in a male bird that is homozygous or heterozygous for the wild-type eye pigment-expression gene on the Z chromosome and/or a female bird that has the wild-type eye pigment-expression gene on the Z chromosome, and/or when the expression level of the eye pigment-expression gene in the biological sample from the test bird is the same as (not significantly different from) the expression level of the eye pigment-expression gene in a male bird that is homozygous for the loss-of-function eye pigment-expression gene on the Z chromosome and/or a female bird that has the loss-of-function eye pigment-expression gene on the Z chromosome, and/or when the expression level of the eye pigment-expression gene in the biological sample from the test bird is lower than the expression level of the eye pigment-expression gene in a male bird that is homozygous for the loss-of-function eye pigment-expression gene on the Z chromosome and/or a female bird that has the loss-of-function eye pigment-expression gene on the Z chromosome, it can be determined that, for example, the test bird does not have the eye pigment expression function.

When the evaluation is conducted based on the expression of the eye pigment-expression protein, the expression of the eye pigment-expression protein can be detected using, for example, a method in which a spectrophotometer is used (e.g., UV absorption method or bicinchoninic acid method), ELISA, western blotting, or the like. The protein-containing extract from the bird can be prepared using common techniques in the art, such as ultrasonication and physical homogenization with a homogenizer. When the expression level of the eye pigment-expression protein in the biological sample from the test bird is the same as (not significantly different from) the expression level of the eye pigment-expression gene protein in a male bird that is homozygous or heterozygous for the wild-type eye pigment-expression gene on the Z chromosome and/or a female bird that has the wild-type eye pigment-expression gene on the Z chromosome, and/or when the expression level of the eye pigment-expression protein in the biological sample from the test bird is (significantly) higher than the expression level of the eye pigment-expression protein in a male bird that is homozygous or heterozygous for the wild-type eye pigment-expression gene on the Z chromosome and/or a female bird that has the wild-type eye pigment-expression gene on the Z chromosome, and/or when the expression level of the eye pigment-expression protein in the biological sample from the test bird is (significantly) higher than the expression level of the eye pigment-expression protein in a male bird that is homozygous for the loss-of-function eye pigment-expression gene on the Z chromosome and/or a female bird that has the loss-of-function eye pigment-expression gene on the Z chromosome, it can be determined that, for example, the test bird has the eye pigment expression function. On the other hand, when the expression level of the eye pigment-expression protein in the biological sample from the test bird is (significantly) lower than the expression level of the eye pigment-expression protein in a male bird that is homozygous or heterozygous for the wild-type eye pigment-expression gene on the Z chromosome and/or a female bird that has the wild-type eye pigment-expression gene on the Z chromosome, and/or when the expression level of the eye pigment-expression protein in the biological sample from the test bird is the same as (not significantly different from) the expression level of the eye pigment-expression protein in a male bird that is homozygous for the loss-of-function eye pigment-expression gene on the Z chromosome and/or a female bird that has the loss-of-function eye pigment-expression gene on the Z chromosome, and/or when the expression level of the eye pigment-expression protein in the biological sample from the test bird is lower than the expression level of the eye pigment-expression protein in a male bird that is homozygous for the loss-of-function eye pigment-expression gene on the Z chromosome and/or a female bird that has the loss-of-function eye pigment-expression gene on the Z chromosome, it can be determined that, for example, the test bird does not have the eye pigment expression function.

In the present disclosure, the eye pigment-expression gene can be present in an RNA form (e.g., mRNA) or DNA form (e.g., cDNA or genome DNA). The DNA may be doublestranded DNA or single-stranded DNA. In the present disclosure, the gene may include an additional sequence such as an untranslated region (UTR).

The loss of the function of the eye pigment-expression gene means a state in which, in a bird having the loss-of-function eye pigment-expression gene, the function of the eye pigment-expression gene (significantly) decreases or is lost to an extent that the eye pigment expression function is lost, compared to a bird (also referred to as a "wild-type bird" hereinafter) having the wild-type eye pigment-expression gene. Specifically, the loss of the function of the eye pigment-expression gene may mean, for example, a state in which the expression level of the mRNA of the eye pigment-expression gene or the protein encoded by the gene (significantly) decreases, or the mRNA of the eye pigment-expression gene or the protein encoded by the gene is not fully expressed, or a state in which the expression level of the mRNA of a functional eye pigment-expression gene or the protein encoded by the eye pigment-expression gene decreases, or the mRNA of a functional eye pigment-expression gene or the protein encoded by the eye pigment-expression gene is not fully expressed. Accordingly, in the present disclosure, the loss of the function of the eye pigment-expression gene may be caused by introducing the loss-of-function mutation into the eye pigment-expression gene, or by introducing a polynucleotide for suppressing the expression of the eye pigment-expression gene. The term "suppressing the expression of the gene" may mean suppression of the transcription of the gene or suppression of the translation to a protein.

In the present disclosure, a bird in which the function of the eye pigment-expression gene is lost can also be referred to as, for example, a "bird having the loss-of-function eye pigment-expression gene". In general, a male bird is homozygous for the sex chromosome and has only the Z chromosome as the sex chromosome, and a female bird is heterozygous for the sex chromosome and has the Z chromosome and the W chromosome. The bird in the present disclosure has, for example, the eye pigment-expression gene on the Z chromosome. The loss of the eye pigment expression caused by the loss of the function of the eye pigment-expression gene is, for example, a recessive (latent) trait. Accordingly, for example, when the bird of the present disclosure is male, the function of the eye pigment-expression gene may be lost on one of the Z chromosomes, but it is preferable that the function of the eye pigment-expression gene is lost on both of the Z chromosomes. Also, for example, when the bird of the present disclosure is female, the function of the eye pigment-expression gene may be lost on the Z chromosome. In the bird in which the function of the eye pigment-expression gene is lost, genes other than the eye pigment-expression gene may also be subjected to modification, alteration, introduction, and/or loss of the function.

The loss of the function of the eye pigment-expression gene can be caused by, for example, introducing mutation, more specifically loss-of-function mutation, into the eye pigment-expression gene. The type of mutation is not particularly limited, and examples thereof include point mutation, splice mutation, missense mutation, nonsense mutation, frameshift mutation, base deletion over a large area (large deletion), and the like. The mutation may cause, for example, partial or entire deletion of the eye pigment-expression gene. The frameshift mutation occurs when the triplet reading frames (codons) are shifted due to base deletion or base insertion. The frameshift mutation has a very high impact on the gene function compared to base-pair substitution mutation. The reason for this is that, when the frameshift mutation occurs, the genetic codes on the downstream side of the position at which the frameshift mutation has been introduced are significantly shifted, which leads to not only changes in amino acids but also positional shifts of the stop codon and the like.

The loss-of-function eye pigment-expression gene is obtained by, for example, introducing mutation such as deletion, substitution, insertion, and/or addition of one or several bases (also referred to as "one or more bases" hereinafter) into the base sequence of the wild-type eye pigment-expression gene. For example, the description of the number of bases in the description of (S2) above can be applied to the "one or more bases", for example, in the base sequence of the eye pigment-expression gene. The frameshift mutation is caused due to, for example, insertion or deletion of 3m+1 bases or 3m+2 bases (m is an integer larger than or equal to 0). The eye pigment expression function is suppressed in the loss-of-function eye pigment-expression gene due to, for example, such mutation.

In the present disclosure, mutation in the eye pigment-expression gene can be caused by, for example, introducing mutation into the target gene in the genome of a target bird using an ordinary method. An introduction target of the mutation may be, for example, an avian cell, and specific examples thereof include a primordial germ cell, an embryonic stem cell (ES cell), and the like. A method for introducing the mutation can be a method such as homologous recombination or a genome-editing technology in which ZFN, TALEN, CRISPR-CAS9, CRISPR-CPF1, or the like is used. The method for introducing the mutation may be a mutagenesis such as a site-directed mutagenesis. The method for introducing the mutation may be, for example, a random mutagenesis. Examples of the random mutagenesis include: irradiation with α-rays, β-rays, γ-rays, X-rays, or the like; chemical treatment with a mutagenic agent such as ethyl methanesulfonate (EMS) or ethinylnitrosourea (ENU); heavy ion beam treatment; and the like. The method for introducing the mutation with the genome editing technology can be conducted with reference to, for example, Example 1 below, and can be the method described in Japanese Patent No. 7113415. Specifically, the introduction of the mutation with the genome editing technology can be conducted by, for example, introducing a protein and a nucleic acid included in the genome editing technology, or a vector that encodes the protein and the nucleic acid. The protein is, for example, a CRISPR (Clustered Regularly Interspaced Short Palindromic Repeats) enzyme, and specific examples thereof include Cas 1, Cas1B, Cas2, Cas3, Cas4, Cas5, Cas6, Cas7, Cas8, Cas9, Cas10, Csy1, Csy2, Csy3, Cse1, Cse2, Csc1, Csc2, Csa5, Csn2, Csm2, Csm3, Csm4, Csm5, Csm6, Cmr1, Cmr3, Cmr4, Cmr5, Cmr6, Csb1, Csb2, Csb3, Csx17, Csx14, Csx10, Csx16, CsaX, Csx3, Csx1, Csx15, Csf1, Csf2, Csf3, Csf4, and the like. Also, the protein may be, for example, a nuclease that is artificially designed based on the CRISPR enzyme. Examples of the nuclease include MAD7, mutant MAD7 (see Japanese Patent No. 7113415, for example), and the like. The above-mentioned nucleic acid may be crRNA and optionally tracrRNA, or a single-stranded nucleic acid obtained by linking crRNA and tracrRNA via a linker. In this case, the nucleic acid is designed such that, for example, a base sequence of crRNA that anneals to the target sequence is complementary to the base sequence encoding the eye pigment-expression gene. One type of the nucleic acid may be used alone, or two or more types of the nucleic acids may be used together. When the genome editing technology is used, large deletion in the base sequence can be induced between the target sequences using, for example, two or more types of nucleic acids, and it is also possible to, for example, delete the entire target gene. The method for introducing the mutation may be a mutagenesis such as a site-directed mutagenesis.

The position of the mutation in the loss-of-function eye pigment-expression gene, that is, the position at which the mutation is introduced into the eye pigment-expression gene, is not particularly limited, and can be located in any region related to the eye pigment-expression gene. Specific examples of the region in which the position of the mutation is located in the loss-of-function eye pigment-expression gene include the expression control region such as the promoter region for the eye pigment-expression gene, the exon region that includes the coding region encoding the protein encoded by the eye pigment-expression gene, and the non-coding region (e.g., intron region, enhancer region, or the like) that does not encode the protein encoded by the eye pigment-expression gene, and the exon region is preferable. Examples of the exon region include the first exon, the second exon, and the like.

Specifically, when the bird is a chicken and the eye pigment-expression gene is the SLC45A2 gene, the region in which the position of the mutation is located in the SLC45A2 gene is, for example, the region from the base at position 545 to the base at position 615 (SEQ ID NO: 3) in the base sequence of SEQ ID NO: 1. The position of the mutation in the SLC45A2 gene corresponds to, for example, the second exon of the chicken SLC45A2 gene. It is preferable to introduce the frameshift mutation or splice mutation at the position of the mutation.

Base Sequence of Region in Which Position of Mutation Is Located in Chicken SLC45A2 Gene (SEQ ID NO: 3)

5'-
AAGCATATTTATTTGACGTCTGCTCTCATGAAGATAAAGAGAAGGGTCTGCATTATCAT
GCCCTGTTTACA-3'

Examples of the loss-of-function bird SLC45A2 gene include polynucleotides (M) and (T) below or genome regions encoding the polynucleotides (M) and (T).

(M) Any of polynucleotides (M1) to (M7) below:
(M1) a polynucleotide having a base sequence of SEQ ID NO: 1;
(M2) a polynucleotide that encodes a protein whose eye pigment expression function is suppressed and that has a base sequence consisting of the base sequence of (M1) above with deletion, substitution, insertion, and/or addition of one or several bases;
(M3) a polynucleotide that encodes a protein whose eye pigment expression function is suppressed and that has a base sequence having 80% or more identity to the base sequence of (M1) above;
(M4) a polynucleotide that encodes a protein whose eye pigment expression function is suppressed and that has a base sequence complementary to a polynucleotide capable of hybridizing to the polynucleotide having the base sequence of (M1) above under a stringent condition;
(M5) a polynucleotide encoding a protein having an amino acid sequence of SEQ ID NO: 2;
(M6) a polynucleotide encoding a protein whose eye pigment expression function is suppressed and that has an amino acid sequence consisting of the amino acid sequence of SEQ ID NO: 2 with deletion, substitution, insertion, and/or addition of one or several amino acids; and
(M7) a polynucleotide encoding a protein whose eye pigment expression function is suppressed and that has an amino acid sequence having 80% or more identity to the amino acid sequence of SEQ ID NO: 2.

In the descriptions of the polynucleotides of (M1) to (M7) above, the wording "eye pigment expression function is suppressed" means, for example, that the eye pigment expression function is significantly suppressed compared to the protein encoded by the polynucleotide (S1) or (S5), and preferably means that the eye pigment expression function is completely lost.

The "one or several" bases in (M2) above refer to, for example, 1 to 326 bases, 1 to 244 bases, 1 to 163 bases, 1 to 81 bases, 1 to 65 bases, 1 to 48 bases, 1 to 32 bases, 1 to 16 bases, 1 to 8 bases, 1 to 5 bases, 1 to 3 bases, 1 or 2 bases, or 1 base, in the base sequence of SEQ ID NO: 1.

In (M3) above, the identity to the base sequence of SEQ ID NO: 1 is, for example, 80% or more, 85% or more, 90% or more, 95% or more, 96% or more, 97% or more, 98% or more, or 99% or more.

In (M4) above, the "polynucleotide capable of hybridizing" is, for example, a polynucleotide that is fully or partially complementary to the polynucleotide having the base sequence of SEQ ID NO: 1. The hybridization can be detected, for example, through various hybridization assays. The description in (S4) above can be applied to the hybridization and stringent condition in (M4) above.

The "one or several" amino acids in (M6) above refer to, for example, 1 to 108 amino acids, 1 to 81 amino acids, 1 to 54 amino acids, 1 to 27 amino acids, 1 to 21 amino acids, 1 to 16 amino acids, 1 to 10 amino acids, 1 to 5 amino acids, 1 to 3 amino acids, 1 or 2 amino acids, or 1 amino acid, in the amino acid sequence of SEQ ID NO: 2.

In (M7) above, the identity to the amino acid sequence of SEQ ID NO: 2 is, for example, 80% or more, 85% or more, 90% or more, 95% or more, 96% or more, 97% or more, 98% or more, or 99% or more.

(T) any of polynucleotides (T1) to (T4) below:
(T1) a polynucleotide having a base sequence consisting of the base sequence of SEQ ID NO: 1 with deletion of the bases from position 545 to position 615;
(T2) a polynucleotide that encodes a protein whose eye pigment expression function is suppressed and that has a base sequence consisting of the base sequence of the polynucleotide of (T1) above with deletion, substitution, insertion, and/or addition of one or several bases;
(T3) a polynucleotide that encodes a protein whose eye pigment expression function is suppressed and that has a base sequence having 80% or more identity to the base sequence of the polynucleotide of (T1) above; and
(T4) a polynucleotide that encodes a protein whose eye pigment expression function is suppressed and that has a base sequence complementary to a polynucleotide capable of hybridizing to the polynucleotide having the base sequence of (T1) above under a stringent condition.

In the descriptions of the polynucleotides of (T1) to (T4) above, the wording "eye pigment expression function is suppressed" means, for example, that the eye pigment expression function is significantly suppressed compared to the protein encoded by the polynucleotide (S1) or (S5), and preferably means that the eye pigment expression function is completely lost.

The "one or several" bases in (T2) above refer to, for example, 1 to 312 bases, 1 to 234 bases, 1 to 156 bases, 1 to 78 bases, 1 to 62 bases, 1 to 46 bases, 1 to 31 bases, 1 to 15 bases, 1 to 7 bases, 1 to 5 bases, 1 to 3 bases, 1 or 2 bases, or 1 base, in the base sequence of (T1) above.

In (T3) above, the identity to the base sequence of (T1) above is, for example, 80% or more, 85% or more, 90% or more, 95% or more, 96% or more, 97% or more, 98% or more, or 99% or more.

In (T4) above, the "polynucleotide capable of hybridizing" is, for example, a polynucleotide that is fully or partially complementary to the polynucleotide having the base sequence of (T1) above. The hybridization can be detected, for example, through various hybridization assays. The description in (S4) above can be applied to the hybridization and stringent condition in (T4) above.

In the present disclosure, when the loss of the function of the eye pigment-expression gene is caused by suppressing the expression of the eye pigment-expression gene, the loss of the function of the eye pigment-expression gene can be caused by, for example, introducing a polynucleotide for suppressing the expression of the eye pigment-expression gene into a target bird. The method for introducing the polynucleotide is not particularly limited, and can be conducted using, for example, RNA interference, antisense RNA, a genome editing technology, or the like. An expression cassette such as an expression vector that includes the polynucleotide can be introduced into a target bird through, for example, microinjection, a polyethylene glycol method, electroporation, a particle gun method, or the like. The target bird may be an egg, a baby bird, an infant bird, a young bird, or an adult bird.

As described above, the bird of the present disclosure is produced by causing loss-of-function mutation in the wild-type eye pigment-expression gene. Accordingly, the bird of the present disclosure can also be called, for example, a "mutant bird". The bird of the present disclosure may be a bird that is a progeny of the "mutant bird" and has loss-of-function mutation in the eye pigment-expression gene. The bird of the present disclosure can also be referred to as, for example, a mutant bird having, in the base sequence of the eye pigment-expression gene, genetic mutation introduced using the above-described method of introducing mutation. The bird of the present disclosure does not encompass a bird obtained only by way of an essentially biological process.

The description of a production method, which will be described later, can be applied to the method for producing the bird of the present disclosure.

### <Portion of Bird>

In another aspect, the present disclosure provides a portion of a bird in which the function of the eye pigment-expression gene on the Z chromosome is lost. The portion of a bird according to the present disclosure is a portion of the bird of the present disclosure.

An example of the portion of a bird is an edible portion of a bird. Specific examples of the portion of a bird include the muscle, the skin, the heart, the liver, the gizzard, the bone, the cartilage, and the like.

### <Bird Egg Population>

In another aspect, the present disclosure provides a bird egg population. The egg population of the present disclosure is a bird egg population, and when a bird inside the egg is a male bird, the male bird is heterozygous for the eye pigment-expression gene on the Z chromosome and has the loss-of-function pigment-expression gene and the wild-type eye pigment-expression gene, and when a bird inside the egg is a female bird, the female bird has the loss-of-function eye pigment-expression gene on the Z chromosome. The description of the bird of the present disclosure can be applied to the bird egg population of the present disclosure.

The bird egg population of the present disclosure can be favorably used in, for example, a bird sexing method, which will be described later. Accordingly, the bird egg population of the present disclosure can also be referred to as, for example, a "bird egg population to be used for the sexing of a bird".

In the bird egg population of the present disclosure, the number of eggs need only be two or more, and the upper limit thereof is not particularly limited. The number of eggs in the egg population is, for example, 10 to 10,000,000, 10 to 1,000,000, or 10 to 100,000. The number of eggs in the egg population can be adjusted in accordance with, for example, the size of a poultry breeding farm or the like.

The bird egg population of the present disclosure includes, for example, fertilized eggs. In the bird egg population of the present disclosure, each egg is, for example, an egg containing an embryo. The bird egg population is a population that includes, for example, eggs that are incubated for 0 to 10 days, 0 to 7 days, 3 to 7 days, 4 to 7 days, 5 to 7 days, or 6 to 7 days after the day on which the egg is laid, which is used as a reference date (0 days after the start of egg incubation). The fertilized eggs are obtained by, for example, performing egg testing using an egg testing machine or the like and removing unfertilized eggs and growth stopping eggs. It is preferable to perform the egg testing 5 to 6 days after the start of egg incubation.

The bird egg population of the present disclosure may be in the state of being contained in a receptacle or a tool such as an egg tool (e.g., a plastic pack, a setter tray, or a tray) or a transport tool (e.g., a container).

### <Sexing Method>

In another aspect, the present disclosure provides a bird sexing method in which the bird of the present disclosure is used. The bird sexing method of the present disclosure includes a step of sexing a bird inside each egg of a bird egg population based on the color of the eyes of the bird. When the bird inside the egg is a male bird, the male bird is heterozygous for the eye pigment-expression gene on the Z chromosome and has the loss-of-function pigment-expression gene and the wild-type eye pigment-expression gene, and when the bird inside the egg is a female bird, the female bird has the loss-of-function eye pigment-expression gene on the Z chromosome. The description of the bird of the present disclosure can be applied to the sexing method of the present disclosure.

The sexing method of the present disclosure may include a step (preparation step) of preparing the egg population by mating a male parent bird with a female parent bird prior to the sexing. The male parent bird has the loss-of-function eye pigment-expression gene and is homozygous for the loss-of-function pigment-expression gene. The female parent bird has the wild-type eye pigment-expression gene. The preparation step can also be referred to as, for example, the mating step. The description of a mating step of a production method, which will be described later, can be applied to the mating step.

As described above, in the sexing step of the present disclosure, a bird inside each egg of the bird egg population is sexed based on the color of the eyes of the bird. The sexing can be conducted, for example, in the same manner as the above-described direct confirmation. The sexing can be conducted through, for example, visual observation or the like. The sexing may be conducted based on a feather in addition to or instead of the eyes. When a feather is used for the sexing, it is preferable to use a bird belonging to a line in which a pigment is expressed in the feathers of a wild-type bird. When the sexing is conducted visually, in the sexing step, for example, a part of the eggshell of each egg may be removed and then be returned to the original position after the sexing is conducted, or the sexing may be conducted using an egg tester as described above. In the sexing, for example, when the color of the eyes of a bird inside the egg is the same as the color of the eyes of a male bird that is homozygous or heterozygous for the wild-type eye pigment-expression gene on the Z chromosome and/or the color of the eyes of a female bird that has the wild-type eye pigment-expression gene on the Z chromosome, it can be determined that, for example, the bird inside the egg is male. On the other hand, when the color of the eyes of a bird inside the egg is the same as the color of the eyes of a male bird that is homozygous for the loss-of-function type of the wild-type eye pigment-expression gene on the Z chromosome and/or the color of the eyes of a female bird that has the loss-of-function eye pigment-expression gene on the Z chromosome, it can be determined that, for example, the bird inside the egg is female. Specifically, in the case where the eye pigment-expression gene is the SLC45A2 gene, when the color of the eyes of the bird inside the egg is blackish, the bird inside the egg can be determined as a male bird, and when the color of the eyes of the bird inside the egg is not blackish (e.g., transparent or reddish), the bird inside the egg can be determined as a female bird.

The egg population subjected to the sexing step is a population that includes, for example, eggs that are incubated for 0 to 10 days, 0 to 7 days, 3 to 7 days, 4 to 7 days, 5 to 7 days, or 6 to 7 days after the day on which the eggs are laid, which is used as a reference date (0 days after the start of egg incubation), and preferably a population that includes eggs that are incubated for 3 to 7 days, 4 to 7 days, 5 to 7 days, or 6 to 7 days.

In the sexing method of the present disclosure, the bird inside the egg that has been sexed in the sexing step may or may not be further raised. When the bird inside the egg is female, it is preferable that, for example, the bird is further raised. When the bird inside the egg is male, it is preferable that, for example, the bird is not raised. The bird raising conditions and the bird raising method can be determined as appropriate in accordance with, for example, the growth stage of the bird and the breed of the bird. When the bird is raised, the bird may be grown to, for example, any growth stage.

Although the egg population is used for the sexing in the sexing method of the present disclosure, the present disclosure is not limited thereto, and, for example, one or more eggs may be used for the sexing. The description of the egg population can be applied to the above-mentioned eggs, except that, for example, the number of the eggs is one or more.

### <First Production Method>

In another aspect, the present disclosure provides a bird production method of the present disclosure in which the bird of the present disclosure is used. The bird production method (also referred to as a "first production method" hereinafter) of the present disclosure includes a step of mating a male bird with a female bird. The male bird is homozygous for the loss-of-function pigment-expression gene on the Z chromosome, and the female bird has the wild-type eye pigment-expression gene on the Z chromosome.

The first production method of the present disclosure can be used to, for example, produce a bird in which the function of the eye pigment-expression gene is lost because the birds of the present disclosure are used in the mating step.

In the mating step, the male bird used as a first parent is not limited as long as it is homozygous for the loss-of-function pigment-expression gene on the Z chromosome. Regarding the loss-of-function pigment-expression gene, the function may be lost in all the cells of the bird, or the function may be lost in the gametes (sperms). Regarding the loss-of-function pigment-expression gene, when the function is lost in the sperms, the above-mentioned bird can also be referred to as, for example, a "chimeric bird".

The first production method of the present disclosure may include a step (selection step) of selecting, prior to the mating, a male bird that is homozygous for the loss-of-function pigment-expression gene on the Z chromosome from target male birds. The selection step can be conducted, for example, in a manner similar to the above-mentioned direct or indirect confirmation. Specifically, the selection may be conducted based on the color of the eyes of the target male bird, or may be conducted using the base sequence of the eye pigment-expression gene as an indicator, or may be conducted using the expression level of the eye pigment-expression gene or the eye pigment-expression protein as an indicator.

When the base sequence of the eye pigment-expression gene is used as an indicator, the loss of the function of the eye pigment-expression gene may be detected in the selection step by, for example, reading the base sequence of the eye pigment-expression gene of the target male bird and comparing the base sequence to the base sequence of the corresponding wild-type or loss-of-function eye pigment-expression gene. The base sequence can be read using, for example, a sequencer. Then, in the selection step, when, for example, the base sequence of the pigment-expression gene of the target male bird is the base sequence of the corresponding wild-type eye pigment-expression gene of the bird into which loss-of-function mutation is introduced, or is the same as the base sequence of the corresponding loss-of-function eye pigment-expression gene of the bird, the male bird is selected as the bird of the present disclosure. The selection conditions will be described below. Regarding the base sequence of the wild-type eye pigment-expression gene, the above-described base sequence of the wild-type eye pigment-expression gene of the bird can be referred to. Regarding the base sequence of the loss-of-function eye pigment-expression gene, the base sequence of the loss-of-function eye pigment-expression gene of the above-described bird can be referred to. The comparison of the base sequences can be conducted using, for example, a base sequence analysis software (e.g., BLAST described above). In the selection step, the region whose base sequence is subjected to the comparison may be the intron region of the eye pigment-expression gene or the exon region of the pigment-expression gene, but the exon region is preferable. When the loss of the function of the eye pigment-expression gene is caused by introducing mutation such as insertion, deletion, and/or substitution of one or more bases into the base sequence of the corresponding wild-type eye pigment-expression gene, the selection step may be conducted using, for example, a primer set, probes, or a combination thereof capable of detecting at least one type of mutation. The primer set and probes can be designed using, for example, common techniques in the art based on the type of mutation.

In the selection step, when, for example, insertion, deletion, and/or substitution of one or more bases is introduced into the wild-type eye pigment-expression gene, the gene may be determined as a loss-of-function gene. Also, in the selection step, when, for example, frameshift mutation is introduced into the wild-type eye pigment-expression gene, the gene may be determined as a loss-of-function gene. Furthermore, in the selection step, when, for example, the wild-type eye pigment-expression gene is partially or completely deleted, the gene may be determined as a loss-of-function gene.

When the expression level of the eye pigment-expression gene is used as an indicator, the loss of the function of the eye pigment-expression gene may be detected in the selection step by, for example, detecting the function of the mRNA of, or the protein encoded by, the eye pigment-expression gene of the target male bird. Furthermore, in the selection step, the loss of the function of the eye pigment-expression gene may be detected by, for example, detecting whether or not the eye pigment-expression gene or the protein encoded by the eye pigment-expression gene is expressed in the test bird, or detecting the expression level of the eye pigment-expression gene or the protein encoded by the eye pigment-expression gene.

When the determination in the selection step is conducted based on the expression of the protein encoded by the eye pigment-expression gene, for example, the expression level of at least one of the eye pigment-expression gene and the protein encoded by the eye pigment-expression gene in the biological sample from the target male bird is measured in the selection step. Then, in the selection step, a bird in which the function of the eye pigment-expression gene is lost (loss-of-function bird) is selected based on the reference value and the expression level of at least one of the eye pigment-expression gene and the protein encoded by the eye pigment-expression gene in the biological sample from the target male bird. Specifically, in the selection step, the loss-of-function bird can be selected from the target male birds by, for example, comparing the reference value and the expression level of at least one of the eye pigment-expression gene and the protein encoded by the eye pigment-expression gene in the biological sample from the target male bird.

The biological sample from the target male bird is not particularly limited, and may be, for example, the individual target male bird or a portion thereof, and the primordial germ cell (PGC), sperm, and the like of the bird are preferable. For example, one type of biological sample or two or more types of biological samples may be used in the selection step.

In the selection step, the expression level of the eye pigment-expression gene can be measured through, for example, semi-quantitative PCR, quantitative PCR, northern blotting, digital PCR, RNA sequencing (RNAseq) or the like. Also, in the selection step, the expression level of the protein encoded by the eye pigment-expression gene can be measured using, for example, a protein quantification method such as a method in which a spectrophotometer is used (e.g., UV absorption method or bicinchoninic acid method), ELISA, western blotting, or the like.

Examples of the reference value above include the expression level of the eye pigment-expression gene or the protein encoded by the eye pigment-expression gene in the wild-type bird, the expression level of the eye pigment-expression gene or the protein encoded by the eye pigment-expression gene in the bird having the loss-of-function eye pigment-expression gene, and the like. When the expression level of the eye pigment-expression gene in the loss-of-function bird is used as the reference value, the loss-of-function bird may be, for example, a bird in which the function of one of the two eye pigment-expression genes located on a pair of chromosomes is lost, namely a heterozygous bird, or a bird in which the function of both of the two eye pigment-expression genes located on a pair of chromosomes is lost, namely a homozygous bird, but the homozygous bird is preferable. The expression level of the eye pigment-expression gene or the protein encoded by the eye pigment-expression gene used as the reference value can be obtained by, for example, measuring the expression level of the eye pigment-expression gene or the protein encoded by the eye pigment-expression gene in a biological sample collected in the same conditions as those for the biological sample from the target male bird in the same manner as in the case of the biological sample from the target male bird. The reference value may be measured, for example, in advance, or simultaneously with the biological sample from the target male bird.

In this case, the method for evaluating if the function of the eye pigment-expression gene of the target male bird is lost is not particularly limited in the selection step, and can be determined as appropriate in accordance with the type of reference value.

Specifically, when the expression level of the eye pigment-expression gene in the biological sample from the target male bird is the same as (not significantly different from) the expression level of the eye pigment-expression gene in a bird that is homozygous for the wild-type eye pigment-expression gene, and/or when the expression level of the eye pigment-expression gene in the biological sample from the target male bird is (significantly) higher than the expression level of the eye pigment-expression gene in a bird that is homozygous for the wild-type eye pigment-expression gene, and/or when the expression level of the eye pigment-expression gene in the biological sample from the target male bird is (significantly) higher than the expression level of the eye pigment-expression gene in the biological sample from a bird that is homozygous or heterozygous for the loss-of-function eye pigment-expression gene, it can be determined that, for example, the function of the eye pigment-expression gene is not lost in the target male bird. On the other hand, when the expression level of the eye pigment-expression gene in the biological sample from the target male bird is (significantly) lower than the expression level of the eye pigment-expression gene in the biological sample from a bird that is homozygous for the wild-type eye pigment-expression gene, and/or when the expression level of the eye pigment-expression gene in the biological sample from the target male bird is the same as (not significantly different from) the expression level of the eye pigment-expression gene in the biological sample from a bird that is homozygous for the loss-of-function eye pigment-expression gene, and/or when the expression level of the eye pigment-expression gene in the biological sample from the target male bird is (significantly) lower than the expression level of the eye pigment-expression gene in the biological sample from a bird that is homozygous or heterozygous for the loss-of-function eye pigment-expression gene, it can be determined that, for example, the function of the eye pigment-expression gene is lost in the target male bird.

When the expression level of the eye pigment-expression protein in the biological sample from the target male bird is the same as (not significantly different from) the expression level of the eye pigment-expression protein in a bird that is homozygous for the wild-type eye pigment-expression gene, and/or when the expression level of the eye pigment-expression protein in the biological sample from the target male bird is (significantly) higher than the expression level of the eye pigment-expression protein in a bird that is homozygous for the wild-type eye pigment-expression gene, and/or when the expression level of the eye pigment-expression protein in the biological sample from the target male bird is (significantly) higher than the expression level of the eye pigment-expression protein in the biological sample from a bird that is homozygous or heterozygous for the loss-of-function eye pigment-expression gene, it can be determined that, for example, the function of the eye pigment-expression gene is not lost in the target male bird. On the other hand, when the expression level of the eye pigment-expression protein in the biological sample from the target male bird is (significantly) lower than the expression level of the eye pigment-expression protein in the biological sample from a bird that is homozygous for the wild-type eye pigment-expression gene, and/or when the expression level of the eye pigment-expression protein in the biological sample from the target male bird is the same as (not significantly different from) the expression level of the eye pigment-expression protein in the biological sample from a bird that is homozygous for the loss-of-function eye pigment-expression gene, and/or when the expression level of the eye pigment-expression protein in the biological sample from the target male bird is (significantly) lower than the expression level of the eye pigment-expression protein in the biological sample from a bird that is homozygous or heterozygous for the loss-of-function eye pigment-expression gene, it can be determined that, for example, the function of the eye pigment-expression gene is lost in the target male bird.

In the selection step, for example, the bird determined as a bird in which the function of the eye pigment-expression gene is lost is selected as a male bird to be used as the first parent.

In the selection step, for example, the genotype of the eye pigment-expression gene may be evaluated based on the expression level of the eye pigment-expression gene. Specifically, it may be evaluated whether the bird is homozygous for the wild-type gene (normal gene), is heterozygous for the eye pigment-expression gene and has the wild-type gene (normal gene) and the loss-of-function gene, or is homozygous for the loss-of-function gene. In this case, the selection step can be conducted using, as the reference values, a value from a bird (wild-type bird) that is homozygous for the wild-type eye pigment-expression gene and a value from a bird (homozygous bird or heterozygous bird) that is homozygous or heterozygous for the loss-of-function eye pigment-expression gene. Specifically, when the expression level of the target gene in the target male bird is equal to the expression level of the target gene in the wild-type bird, the heterozygous bird, or the homozygous bird in the selection step, it can be determined that, for example, the target male bird has the same genotype as that of the bird with the equal expression level. Accordingly, in the selection step, a male bird that is homozygous for the loss-of-function gene can be selected as the above-mentioned male bird.

In the first production method of the present disclosure, it is preferable that the male bird selected in the selection step is further raised in order to use the bird in the mating step. The bird raising conditions and the bird raising method can be determined as appropriate in accordance with, for example, the growth stage of the bird and the breed of the bird. When the bird is raised, the bird may be grown to, for example, any growth stage.

The first production method of the present disclosure may also include a collection step of collecting sperms from the male bird obtained in the selection step.

The first production method of the present disclosure may include a step (breeding step) of breeding, prior to the mating, a male bird that is homozygous for the loss-of-function pigment-expression gene on the Z chromosome from target male birds. The breeding step can also be referred to as, for example, a step (functional loss causing step) of causing the loss of the function of the eye pigment gene of the target male bird.

The breeding step may be conducted by introducing loss-of-function mutation into the eye pigment-expression gene, or introducing a polynucleotide for suppressing the expression of the eye pigment-expression gene, or mating a male bird that is homozygous for the loss-of-function pigment-expression gene on the Z chromosome with a female bird having the loss-of-function pigment-expression gene on the Z chromosome, that is, crossing the loss-of-function eye pigment-expression gene.

When introduced into the eye pigment-expression gene, loss-of-function mutation is introduced into, for example, the eye pigment-expression gene of the target bird in the functional loss causing step. The target bird has the eye pigment-expression gene on the Z chromosome. Accordingly, in the functional loss causing step, for example, the loss of the function of the eye pigment-expression gene may be caused on one of the pair of Z chromosomes of the target male bird, or the loss of the function of the eye pigment-expression gene is caused on both of the Z chromosomes, but the latter is preferable. In the former case (i.e., when the loss of the function of the eye pigment-expression gene is caused on one of the Z chromosomes), it is preferable that, in the functional loss causing step, the obtained male bird is subjected to crossing to produce a male bird that is homozygous for the loss-of-function gene on the Z chromosome. Also, in the functional loss causing step, for example, the loss of the function of the eye pigment-expression gene on the single Z chromosome of the target female bird may be caused. In this case, it is preferable that, in the functional loss causing step, the obtained female bird is subjected to crossing to produce a male bird that is homozygous for the loss-of-function gene on the Z chromosome.

The loss of the function of the eye pigment-expression gene can be caused by, for example, introducing mutation as described above. The description above can be applied to the mutation, and nonsense mutation or frameshift mutation is preferable. The loss-of-function mutation may be introduced by, for example, introducing deletion, substitution, insertion, and/or addition of one or several bases into the gene (the base sequence of the gene), and it is preferable that the loss-of-function mutation is introduced through partial or complete deletion of the wile-type eye pigment-expression gene.

In the functional loss causing step, the region of the eye pigment-expression gene into which loss-of-function mutation is introduced may be the intron region of the eye pigment-expression gene or the exon region of the pigment-expression gene, but the exon region is preferable.

The loss of the function of the eye pigment-expression gene can be caused by, for example, introducing mutation into the eye pigment-expression gene using an ordinary method. A method for introducing the mutation can be a method such as homologous recombination or a genome-editing technology in which ZFN, TALEN, the above-described CRISPR enzyme (e.g., CRISPR-CAS9 or CRISPR-CPF1), or the like is used. The method for introducing the mutation with the genome editing technology can be conducted with reference to, for example, Example 1 below. Specifically, gene mutation can be introduced into the primordial germ cell (PGC) of the bird or the ES cell, using the genome editing technology. The method for introducing the mutation may be, for example, a random mutagenesis. Examples of the random mutagenesis include: irradiation with α-rays, β-rays, y-rays, X-rays, or the like; chemical treatment with a mutagenic agent such as ethyl methanesulfonate (EMS) or ethinylnitrosourea (ENU); heavy ion beam treatment; and the like. Note that the above-described methods for introducing mutation may be conducted using, for example, commercially available kits and the like.

When the polynucleotide for suppressing the expression of the eye pigment-expression gene is introduced, the method for introducing the polynucleotide is not particularly limited, and can be conducted using, for example, RNA interference, antisense RNA, or a genome editing technology. An expression cassette such as an expression vector that includes the polynucleotide can be introduced into a target bird through, for example, microinjection, a polyethylene glycol method, electroporation, a particle gun method, or the like. The target bird may be, for example, a primordial germ cell, an ES cell, an egg, an embryo, a baby bird, an infant bird, a young bird, or an adult bird, but the primordial germ cell or ES cell is preferable.

It is preferable that, in the first production method of the present disclosure, cells in which loss-of-function mutation is introduced into the eye pigment-expression gene are selected after the introduction of the mutation. The selection can be conducted, for example, in the same manner as the above-described selection step, and the description of the selection step can be applied thereto.

After the introduction of the mutation, for example, the cells into which the mutation has been introduced may be transplanted to the target male bird using an ordinary method. Examples of the transplantation include transplantation to a blastoderm of an early embryo, the blood, a gonadal region, and the like. Before the transplantation, the cells into which the mutation has been introduced may be cultured.

After the transplantation, it is preferable that the target male bird is further raised in order to use the bird in the mating step. The bird raising conditions and the bird raising method can be determined as appropriate in accordance with, for example, the growth stage of the bird and the breed of the bird. When the bird is raised, the bird may be grown to, for example, any growth stage.

The first production method of the present disclosure may also include a collection step of collecting sperms from the male bird obtained in the breeding step.

The first production method of the present disclosure may also include, for example, a step of identifying the genotype of the eye pigment-expression gene of the progeny.

### <Second Production Method>

In another aspect, the present disclosure provides a bird production method in which the bird of the present disclosure is used. The bird production method (also referred to as a "second production method" hereinafter) of the present disclosure includes a step of mating a male bird with a female bird, wherein the male bird and/or the female bird is the bird of the present disclosure. With the bird production method of the present disclosure, it is possible to maintain progenies of the bird of the present disclosure.

In the second production method of the present disclosure, it is sufficient that the bird of the present disclosure is used as at least one of the male bird and the female bird, and the other bird may be the bird of the present disclosure, or a bird that is homozygous or heterozygous for the wild-type eye pigment gene.

The second production method of the present disclosure may also include, for example, a step of identifying the genotype of the eye pigment gene of the progeny. The second production method of the present disclosure may include, for example, a step of selecting a male bird that is homozygous for the loss-of-function pigment-expression gene based on the genotype.

### <Third Production Method>

In another aspect, the present disclosure provides a bird production method in which the bird that has been sexed using the sexing method of the present disclosure is used. The bird production method (also referred to as a "third production method" hereinafter) of the present disclosure includes a step of sexing a bird inside each egg of a bird egg population based on the color of the eyes of the bird, and a step of obtaining a progeny using the bird that has been sexed, wherein the sexing is conducted using the bird sexing method of the present disclosure. With the third production method of the present disclosure, it is possible to easily maintain parent birds for use in production of an egg population subjected to the sexing method of the present disclosure.

The progeny is obtained by, for example, mating the bird that has been sexed with another bird. When the bird that has been sexed is a male bird, the mating can be conducted by, for example, mating the male bird that has been sexed with a female bird. The female bird may be, for example, the bird of the present disclosure, or a bird that is homozygous or heterozygous for the wild-type eye pigment-expression gene. Also, when the bird that has been sexed is a female bird, the mating can be conducted by, for example, mating the female bird that has been sexed with a male bird. The male bird may be, for example, the bird of the present disclosure, or a bird that is homozygous or heterozygous for the wild-type eye pigment-expression gene.

The third production method of the present disclosure may also include, for example, a step of identifying the genotype of the eye pigment-expression gene of the progeny. The third production method of the present disclosure may include, for example, a step of selecting a male bird that is homozygous for the loss-of-function pigment-expression gene based on the genotype.

### <Sexing Apparatus>

In another aspect, the present disclosure provides a sexing apparatus capable of sexing the egg population of the present disclosure. The sexing apparatus of the present disclosure is a sexing apparatus including: a light source for irradiating a sexing target egg selected from a bird egg population with light; an imaging unit for imaging the sexing target egg in a state in which a content thereof is visible due to irradiation with light from the light source and outputting image data containing an eye of a bird inside the sexing target egg; and a sexing unit for sexing the bird inside the sexing target egg based on the image data. With the sexing apparatus of the present disclosure, it is possible to easily sex, for example, the egg population of the present disclosure.

Hereinafter, examples of the sexing apparatus of the present disclosure will be described with reference to the drawings. However, the sexing apparatus of the present disclosure is not limited to the following examples, and can be implemented with any modifications.

### (Embodiment of Sexing Apparatus)

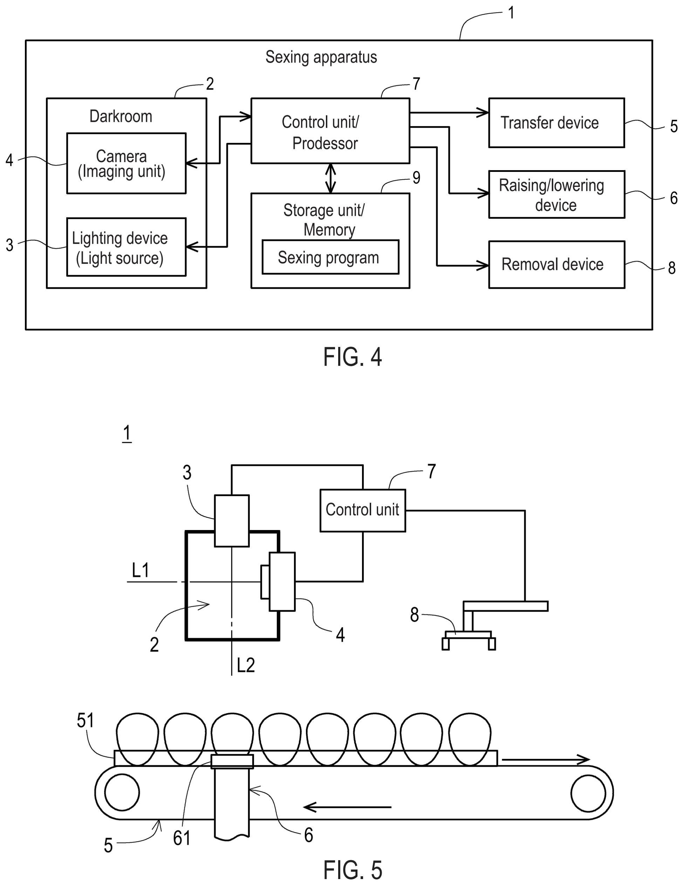

A sexing apparatus according to an embodiment of the present disclosure will be described with reference to FIGS. 4 to 8. In the following description, a sexing apparatus 1 illustrated in FIGS. 4 to 6 is shown as an example of the sexing apparatus according to the present disclosure. The sexing apparatus 1 is an apparatus for sexing one egg (also referred to merely as a "sexing target egg" hereinafter) selected from a plurality of eggs included in the above-described "bird egg population".

As shown in FIGS. 4 to 6, the sexing apparatus 1 includes a darkroom 2, a lighting device 3, a camera 4, a transfer device 5, a raising/lowering device 6, a control unit 7, a removal device 8, and a storage unit 9.

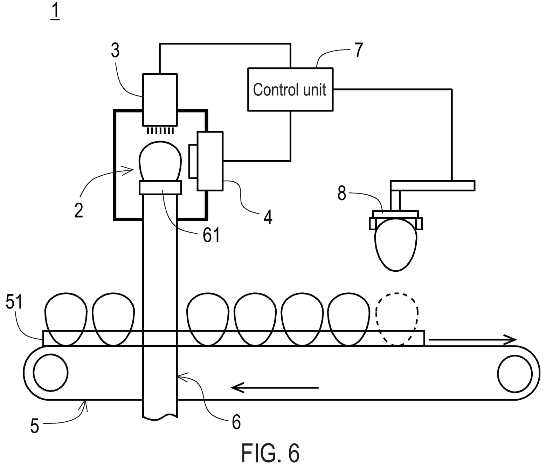

The darkroom 2 provides a space for imaging the sexing target egg using the camera 4 with light being blocked. By disposing the sexing target egg in the darkroom 2 during the imaging, it is possible to suppress external perturbations during the imaging in the sexing apparatus 1. As shown in FIG. 4, the lighting device 3 and the camera 4 are provided in the darkroom 2. Also, as shown in FIG. 6, the raising/lowering device 6 for moving the sexing target egg to the darkroom 2 is configured such that a portion thereof is capable of being inserted into and removed from the darkroom 2.

The lighting device 3 is an example of a "light source" of the present disclosure and is composed of an LED light or the like. The lighting device 3 is used to irradiate the sexing target egg with light during imaging of the sexing target egg using the camera 4. As shown in FIG. 5, the lighting device 3 is disposed at a position where an optical axis L1 of the camera 4 and an optical axis L2 of the lighting device 3 intersect each other. In the example shown in FIG. 5, the lighting device 3 is installed at a position in the darkroom 2 where light is emitted from the ceiling side.

The camera 4 is an example of an "imaging unit" of the present disclosure and is used to image the sexing target egg. The camera 4 is composed of, for example, a color CCD camera, a color CMOS camera, or the like. The camera 4 images the sexing target egg in a state in which the content thereof is visible due to irradiation with light from the lighting device 3, and outputs image data containing the eye of a bird inside the sexing target egg. Note that the image data outputted by the camera 4 are inputted into the control unit 7, which will be described below.

Subsequently, the transfer device 5 and the raising/lowering device 6, which are examples of a "transport unit" of the present disclosure, will be described.

The transfer device 5 is a device for transferring a plurality of eggs (bird egg population) to be sexed placed on a dedicated tray 51. As shown in FIG. 5, each of the plurality of eggs is placed on the dedicated tray 51 such that the blunt end (air chamber side) faces upward and the sharp end faces downward.

The transfer device 5 drives a conveyor and thus transfers a plurality of eggs placed on the dedicated tray 51 to a position of a support table 61 of the raising/lowering device 6 below the darkroom 2. In the example shown in FIG. 5, the plurality of eggs placed on the dedicated tray 51 are transferred from the left side of the diagram (the upstream side in the transfer direction) to the right side of the diagram (the downstream side in the transfer direction). When the sexing target egg in the plurality of eggs is positioned right above the support table 61 of the raising/lowering device 6, the transfer device 5 stops the conveyor once and thus stops the transfer.

The raising/lowering device 6 raises the support table 61 and thus moves the sexing target egg from the dedicated tray 51 to the support table 61. Thereafter, as shown in FIG. 6, the raising/lowering device 6 further raises the support table 61 and moves the sexing target egg placed on the support table 61 into the darkroom 2. Note that the sexing target egg moved to the support table 61 is continuously maintained to be in the state in which the blunt end (air chamber side) faces upward and the sharp end faces downward.

As shown in FIG. 6, in the darkroom 2, the blunt end (air chamber side) of the sexing target egg comes close to the lighting device 3. During the imaging of the sexing target egg using the camera 4, the lighting device 3 irradiates the sexing target egg with light from the blunt end side. When the sexing target egg is irradiated with light from the blunt end side, the content of the sexing target egg is visible as described above. Thus, the camera 4 can image a bird inside the sexing target egg.

When the imaging of the sexing target egg using the camera 4 in the darkroom 2 is completed, the raising/lowering device 6 lowers the support table 61 and returns the sexing target egg to the dedicated tray 51 of the transfer device 5. Then, the transfer device 5 drives the conveyor again and transfers a sexing target egg to be subsequently sexed to a position right above the support table 61. In this manner, sexing target eggs are successively moved into the darkroom 2 and imaged using the camera 4.

The raising/lowering device 6 includes a rotating mechanism (an example of a "rotating unit" according to the present disclosure) for rotating the support table 61 about a rotation axis extending in the raising/lowering direction. As described above, the camera 4 outputs image data containing the eye of a bird inside the sexing target egg. However, depending on the position (orientation) of the bird inside the sexing target egg, there is a possibility that the image data from the camera 4 do not contain the eye of the bird. In this case, the bird cannot be sexed.

Accordingly, when the control unit 7 cannot detect the eye of the bird in the image data, the raising/lowering device 6 drives the rotation mechanism and thus rotates the support table 61 about the rotation axis extending in the raising/lowering direction. Thereafter, imaging using the camera 4 is conducted again. That is to say, in this embodiment, imaging is repeated until the eye of the bird is detected in image data from the camera 4. The sexing target egg is placed on the support table 61 in the state in which the blunt end (air chamber side) faces upward and the sharp end faces downward. That is to say, the following expression is also possible: the rotating mechanism of the raising/lowering device 6 rotates about the rotation axis extending along a straight line connecting the sharp end and the blunt end of the sexing target egg.

The control unit 7 is configured as a computer system that includes one or more hardware processors (e.g., CPU (Central Processing Unit) and GPU (Graphics Processing Unit)) and the like. The control unit 7 realizes the sexing processing by executing, in the CPU or the like, a predetermined software program (an example of a "sexing program" of the present disclosure) stored in the storage unit 9.

The control unit 7 functions as a control unit for exercising general control of all the operations of the lighting device 3, the camera 4, the transfer device 5, the raising/lowering device 6 and the removal device 8 in the sexing processing by executing the above-described sexing program. Also, the control unit 7 executes the sexing processing for sexing a bird inside the sexing target egg based on the image data containing the eye of the bird inside the sexing target egg imaged using the camera 4. That is to say, the control unit 7 functions as a "sexing unit" of the present disclosure.

The storage unit 9 is composed of a storage device such as a hard disk drive (HDD) and/or a solid state drive (SSD). The storage unit 9 stores the above-described sexing program and the like.

The removal device 8 is disposed on the downstream side in the transfer direction (the right side of the diagram) relative to the darkroom 2. As shown in FIG. 6, the removal device 8 removes, from the dedicated tray 51, a sexing target egg whose sex has been determined as a "male" by the control unit 7. Thus, eggs of male birds are eliminated from a bird egg population, and a female bird egg population is obtained.

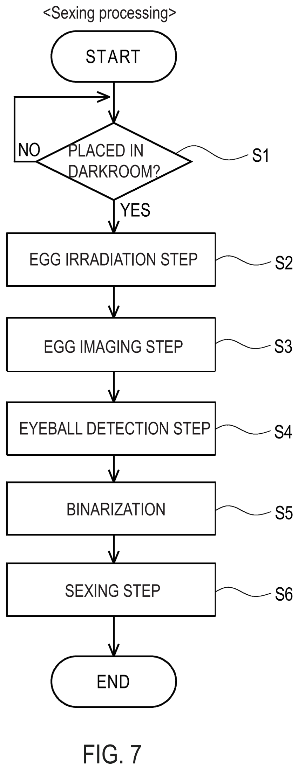

Subsequently, the sexing processing executed by the control unit 7 will be described in detail with reference to the flowchart shown in FIG. 7.

First, when the sexing target egg is disposed inside darkroom 2 by the raising/lowering device 6 (YES in step S1), the control unit 7 operates the lighting device 3 and allows the lighting device 3 to irradiate the sexing target egg with light from the blunt end (air chamber side) (step S2).

Thereafter, the control unit 7 operates the camera 4 and allows the camera 4 to image the sexing target egg in a state in which the content thereof is visible due to irradiation with light from the lighting device 3 and output image data containing the eye of a bird inside the sexing target egg (step S3).

Next, the control unit 7 executes the sexing processing for sexing the bird inside the sexing target egg based on the outputted image data.

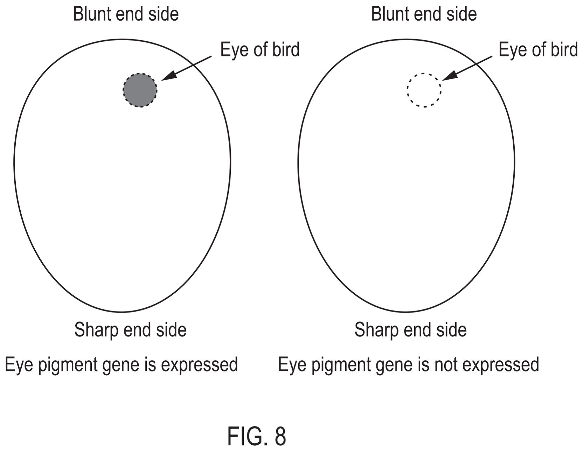

Specifically, the control unit 7 executes eyeball image detection processing for detecting the eye (specifically an eyeball image) of the bird in the images corresponding to the image data (step S4). Particularly, the control unit 7 detects a broken line region shown in FIG. 8 in the images based on the image data through the eyeball image detection processing. Note that known eyeball image detection processing as described in JP 2007-081992A is used as the eyeball image detection processing. When the blunt end (air chamber side) of the egg faces upward, the eyes of the bird are located on the same side as the blunt end. Therefore, the control unit 7 may execute the eyeball image detection processing for detecting the eye of a bird at a predetermined position of the egg image in the image data.

Next, the control unit 7 executes binarization on the detected eyeball image (broken line region), and converts the detected eyeball image to a black-and-white image (step S5). Here, the binarization is an example of a "predetermined image processing" of the present disclosure.

Then, the control unit 7 determines the sex of the bird as "male" when a "black image" makes up a larger region in the binarized black-and-white image, whereas the control unit 7 determines the sex of the bird as "female" when a "white image" makes up a larger region (step S6). Here, the case where a "black image" makes up a larger region is an example of a first condition of the present disclosure, and the case where a "white image" makes up a larger region is an example of a second condition of the present disclosure.

With the sexing apparatus 1 of the present disclosure, it is possible to sex a bird.

### (Modified Examples of Sexing Apparatus)

The sexing apparatus of the present disclosure is not limited to the embodiment above, and various modifications and improvements can be made within the scope described in the claims.

For example, the case where a bird is sexed through detection and binarization of an eyeball image is described as an example in the embodiment above, but there is no limitation thereto, and a bird may be sexed using a previously constructed trained model.

Specifically, a considerable number of images of chicken eyes 7 days after the start of egg incubation are prepared as image data, and each of the images is labeled with "male" or "female" to prepare training data. A trained model M1 is constructed by subjecting the prepared training data to machine learning such as deep learning using multi-layered deep neural network as a model.

Similarly, a considerable number of images of chicken eyes 10 days after the start of egg incubation are prepared as image data, and each of the images is labeled with "male" or "female" to prepare training data. A trained model M2 is constructed by subjecting the prepared training data to machine learning in the same manner.

The constructed trained models M1 and M2 are stored in the storage unit 9.

When the sexing target egg 7 days after the start of egg incubation is imaged using the camera 4, the control unit 7 acquires the trained model M1 in the memory from the storage unit 9 and sexes a bird inside the sexing target egg. Also, when the sexing target egg 10 days after the start of egg incubation is imaged using the camera 4, the control unit 7 acquires the trained model M2 in the memory from the storage unit 9 and sexes a bird inside the sexing target egg. As described above, the sexing apparatus of the present disclosure can sex a bird using the trained models M1 and M2 constructed through machine learning.

Also, for example, the lighting device 3 irradiates the sexing target egg with light from the blunt end side in the embodiment above, but the lighting device 3 may irradiate the sexing target egg with light from the sharp end side or lateral side such that the content thereof is visible.

Furthermore, for example, the camera 4 is disposed at a position where the optical axis L1 of the camera 4 and the optical axis L2 of the lighting device 3 intersect each other in the embodiment above, but the camera 4 may be configured to be capable of taking similar images through a mirror system. In this case, the camera 4 can be disposed at a position where the optical axis L1 of the camera 4 changed by the mirror and the optical axis L2 of the lighting device 3 intersect each other.

### Examples

Hereinafter, the present disclosure will be described in detail by way of examples, but the present disclosure is not limited to aspects described in the examples.

### [Example 1]

A male chicken whose primordial germ cell was homozygous for the loss-of-function SLC45A2 gene was produced and mated with a wild-type female chicken, and it was checked whether a bird inside an egg obtained thorough the mating can be sexed based on the color of the eyes of the bird inside the egg.

The gRNA (SEQ ID NO: 4) of the chicken SLC45A2 gene was cloned into an MAD7 (ST7) expression vector optimized for the gene editing of the primordial germ cell (PGC). After the cloning, the chicken PGCs were obtained from the E3 (embryonic day 3) embryo of a chicken belonging to the BPR (Barred Plymouth Rock) line under the culture conditions described in Reference 2. After the PGCs were obtained, the cloned gRNA was introduced into the PGC through electroporation using the Neon Transfection System (manufactured by Thermo Fisher Scientific).

Reference 2: Chen, Y.C., Lin, S.P., Chang, Y.Y., Chang, W.P., Wei, L.Y., Liu, H.C., Huang, J.F., Pain, B., and Wu, S.C. (2019). In vitro culture and characterization of duck primordial germ cells. Poult Sci 98, 1820-1832. 10.3382/ps/pey515.

gRNA of Chicken SLC45A2 Gene (SEQ ID NO: 4)
5'- TTTGACGTCTGCTCTCATGAAGATA 3'

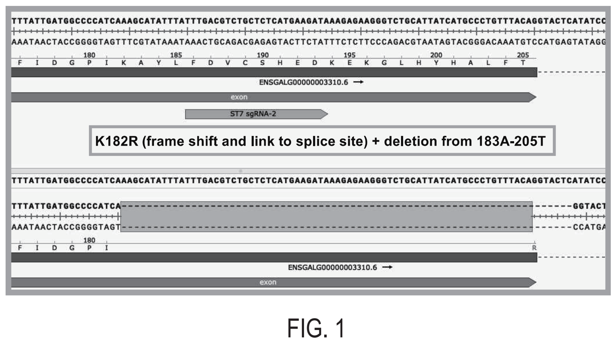

Fluorescent cell enrichment sorting was performed using a BD FACSMelody cell sorter (manufactured by BD Biosciences) two days after the electroporation. In addition, cell culture of the PGC population obtained through the sorting was performed for 1 week in order to isolate an SLC45A2-KO clone. After the culture, the cells were distributed using a cell sorter such that a single cell was contained in one well of a 96-well plate. Then, the obtained clone was subjected to Sanger sequencing to determine the genotype of the target region in the SLC45A2 gene. FIG. 1 shows the results.

FIG. 1 is a diagram showing the genotype of the target region of the SLC45A2 gene of the obtained clone. As shown in FIG. 1, it was revealed that, in the second exon of the SLC45A2 gene, the amino acid at position 182, lysine (K), was substituted with arginine (R), and a region from the amino acid at position 183, alanine (A), to the amino acid at position 205, threonine (T), was deleted. These results suggest that a clone in which the function of SLC45A2 gene was lost due to frameshift mutation and/or splice mutation could be produced.

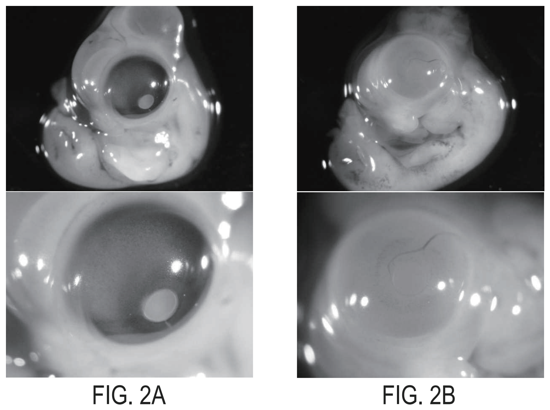

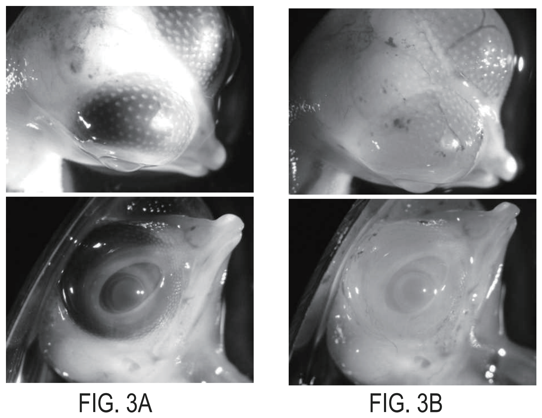

After the genotype was determined, 5×10⁴ PGCs of the SLC45A2-KO clone in total were transplanted into the circulatory system of an E3 recipient chicken to produce a chimeric chicken (F0). Next, an offspring (F1) of the SLC45A2-KO chicken was produced. Specifically, a brood wild-type female chicken was artificially inseminated with sperms collected from the sexually mature male chimeric chicken. After the artificial insemination, an F1 chicken egg population was obtained. The eye pigment of chickens inside the eggs was checked. FIGS. 2 and 3 show the results.

FIG. 2 shows photographs illustrating the color of the eye of the chicken 7 days after the start of egg incubation. In FIG. 2(A), the upper photograph illustrates the entire body of the observed male chicken, and the lower photograph illustrates the eye of the observed male chicken. In FIG. 2(B), the upper photograph illustrates the entire body of the observed female chicken, and the lower photograph illustrates the eye of the observed female chicken. FIG. 3 shows photographs illustrating the color of the eye of the chicken 10 days after the start of egg incubation. In FIG. 3(A), the upper photograph illustrates the top of the head and the eyes of the observed male chicken, and the lower photograph illustrates the right eye of the observed male chicken. In FIG. 3(B), the upper photograph illustrates the top of the head and the eyes of the observed female chicken, and the lower photograph illustrates the right eye of the observed female chicken. As shown in FIGS. 2 and 3, the eyes of the male chicken were black, whereas the eyes of the female chicken were not black, 7 and 10 days after the start of egg incubation. It was found from these results that the eye pigment was expressed in the male chicken obtained using the method of the present disclosure, and the eye pigment expression was suppressed in the female chicken obtained using the method of the present disclosure. It was also found that, when the chicken obtained using the method of the present disclosure was female, the eye pigment expression was suppressed by introducing the loss-of-function mutation into the SLC45A2 gene, and thus the color of the eyes changed (became transparent). Accordingly, it can be said that, with the sexing method of the present disclosure, evaluation can be conducted using an optical approach with an egg tester or the like. Furthermore, it was found that, with the sexing method of the present disclosure, a chicken can be sexed 7 days after the start of egg incubation.

As described above, the present disclosure has been described with reference to the embodiments and the examples, but the present disclosure is not limited to the above-described embodiments and examples. Various modifications that can be understood by a person skilled in the art can be made in the configurations and details of the present disclosure without departing from the scope of the present disclosure.

The contents of the patents, patent applications, and references cited herein are incorporated herein by reference in their entireties as if specifically set forth herein.

The present application claims the benefit of priority from Japanese Patent Application No. 2023-078539 filed on May 11, 2024, the entire disclosure of which is incorporated herein.

### <Supplementary Notes>

Some or all of the embodiments and the examples given above can be described as in the following supplementary notes. However, the scope of the present disclosure is not limited thereto.

### <Sexing Method>

### (Supplementary Note 1)

A bird sexing method including a step of sexing a bird inside each egg of a bird egg population based on a color of eyes of the bird,
wherein when the bird inside the egg is a male bird, the male bird is heterozygous for an eye pigment-expression gene on a Z chromosome and has a loss-of-function pigment-expression gene and a wild-type eye pigment-expression gene, and
when the bird inside the egg is a female bird, the female bird has a loss-of-function eye pigment-expression gene on a Z chromosome.

### (Supplementary Note 2)

The sexing method according to Supplementary Note 1, including a step of preparing the egg population by mating a male parent bird with a female parent bird prior to the sexing,
wherein the male parent bird has the loss-of-function eye pigment-expression gene, and is homozygous for the loss-of-function pigment-expression gene, and
the female parent bird has the wild-type eye pigment-expression gene.

### (Supplementary Note 3)

The sexing method according to Supplementary Note 1 or 2,
wherein the loss-of-function pigment-expression gene is a mutant gene having a base sequence consisting of the base sequence of the wild-type eye pigment-expression gene with deletion, substitution, insertion, and/or addition of one or several bases, and its eye pigment expression function is suppressed.

### (Supplementary Note 4)

The sexing method according to Supplementary Note 3,
wherein the loss-of-function pigment-expression gene is a mutant gene having a base sequence consisting of the base sequence of the wild-type eye pigment-expression gene with deletion of at least some bases, and its eye pigment expression function is suppressed.

### (Supplementary Note 5)

The sexing method according to any one of Supplementary Notes 1 to 4,
wherein the loss-of-function pigment-expression gene is a mutant gene having a base sequence consisting of the base sequence of the wild-type eye pigment-expression gene with frameshift mutation, and its eye pigment expression function is suppressed.

### (Supplementary Note 6)

The sexing method according to any one of Supplementary Notes 1 to 5,
wherein the loss-of-function pigment-expression gene is a mutant gene having a base sequence consisting of the base sequence of the wild-type eye pigment-expression gene with splice mutation, and its eye pigment expression function is suppressed.

### (Supplementary Note 7)

The sexing method according to any one of Supplementary Notes 1 to 6,
wherein the eye pigment-expression gene is an SLC45A2 gene.

### (Supplementary Note 8)

The sexing method according to any one of Supplementary Notes 1 to 7,
wherein the eye pigment-expression gene is an SLC45A2 gene, and
a loss-of-function SLC45A2 gene is a mutant gene of a wild-type SLC45A2 gene having mutation in a second exon, and its eye pigment expression function is suppressed.

### (Supplementary Note 9)

The sexing method according to Supplementary Note 8,
wherein a base sequence of SEQ ID NO: 3 in the second exon of the wild-type SLC45A2 gene is deleted in the loss-of-function SLC45A2 gene.

### (Supplementary Note 10)

The sexing method according to any one of Supplementary Notes 7 to 9,
wherein the SLC45A2 gene is a gene that includes a polynucleotide (S) below:
(S) any of polynucleotides (S1) to (S7) below:
(S1) a polynucleotide having a base sequence of SEQ ID NO: 1;
(S2) a polynucleotide that encodes a protein having an eye pigment expression function and that has a base sequence consisting of the base sequence of (S1) above with deletion, substitution, insertion, and/or addition of several bases;
(S3) a polynucleotide that encodes a protein having an eye pigment expression function and that has a base sequence having 90% or more identity to the base sequence of (S1) above;
(S4) a polynucleotide that encodes a protein having an eye pigment expression function and that has a base sequence complementary to a polynucleotide capable of hybridizing to the polynucleotide having the base sequence of (S1) above under a stringent condition;
(S5) a polynucleotide encoding a protein having an amino acid sequence of SEQ ID NO: 2;
(S6) a polynucleotide encoding a protein that has an eye pigment expression function and that has an amino acid sequence consisting of the amino acid sequence of SEQ ID NO: 2 with deletion, substitution, insertion, and/or addition of several amino acids; and
(S7) a polynucleotide encoding a protein that has an eye pigment expression function and that has an amino acid sequence having 90% or more identity to the amino acid sequence of SEQ ID NO: 2.

### (Supplementary Note 11)

The sexing method according to any one of Supplementary Notes 7 to 10,
wherein the loss-of-function pigment-expression gene is a loss-of-function SLC45A2 gene, and
the loss-of-function SLC45A2 gene is a gene that includes a polynucleotide (M) below:
   (M) any of polynucleotides (M1) to (M7) below:
   (M1) a polynucleotide having a base sequence of SEQ ID NO: 1;
   (M2) a polynucleotide that encodes a protein whose eye pigment expression function is suppressed and that has a base sequence consisting of the base sequence of (M1) above with deletion, substitution, insertion, and/or addition of several bases;
   (M3) a polynucleotide that encodes a protein whose eye pigment expression function is suppressed and that has a base sequence having 80% or more identity to the base sequence of (M1) above;
   (M4) a polynucleotide that encodes a protein whose eye pigment expression function is suppressed and that has a base sequence complementary to a polynucleotide capable of hybridizing to the polynucleotide having the base sequence of (M1) above under a stringent condition;
   (M5) a polynucleotide encoding a protein having an amino acid sequence of SEQ ID NO: 2;
   (M6) a polynucleotide encoding a protein whose eye pigment expression function is suppressed and that has an amino acid sequence consisting of the amino acid sequence of SEQ ID NO: 2 with deletion, substitution, insertion, and/or addition of several amino acids; and
   (M7) a polynucleotide encoding a protein whose eye pigment expression function is suppressed and that has an amino acid sequence having 80% or more identity to the amino acid sequence of SEQ ID NO: 2.

### (Supplementary Note 12)

The sexing method according to any one of Supplementary Notes 7 to 11,
wherein the loss-of-function pigment-expression gene is a loss-of-function SLC45A2 gene, and
the loss-of-function SLC45A2 gene is a gene that includes a polynucleotide (T) below:
   (T) any of polynucleotides (T1) to (T4) below:
   (T1) a polynucleotide having a base sequence consisting of the base sequence of SEQ ID NO: 1 with deletion of the bases from position 545 to position 615;
   (T2) a polynucleotide that encodes a protein whose eye pigment expression function is suppressed and that has a base sequence consisting of the base sequence of the polynucleotide of (T1) above with deletion, substitution, insertion, and/or addition of several bases;
   (T3) a polynucleotide that encodes a protein whose eye pigment expression function is suppressed and that has a base sequence having 80% or more identity to the base sequence of the polynucleotide of (T1) above; and
   (T4) a polynucleotide that encodes a protein whose eye pigment expression function is suppressed and that has a base sequence complementary to a polynucleotide capable of hybridizing to the polynucleotide having the base sequence of (T1) above under a stringent condition.

### (Supplementary Note 13)

The sexing method according to any one of Supplementary Notes 1 to 12,
wherein the bird is selected from the group consisting of a chicken, a quail, a turkey, a domestic duck, a goose, an ostrich, a dove, a guinea fowl, a pheasant, a duck, and an emu.

### <Bird>

### (Supplementary Note 14)

A bird having a loss-of-function eye pigment-expression gene on a Z chromosome.

### (Supplementary Note 15)

The bird according to Supplementary Note 14,
wherein the loss-of-function pigment-expression gene is a mutant gene having a base sequence consisting of a base sequence of a wild-type eye pigment-expression gene with deletion, substitution, insertion, and/or addition of one or several bases, and its eye pigment expression function is suppressed.

### (Supplementary Note 16)

The bird according to Supplementary Note 15,
wherein the loss-of-function pigment-expression gene is a mutant gene having a base sequence consisting of the base sequence of the wild-type pigment-expression gene with deletion of at least some bases, and its eye pigment expression function is suppressed.

### (Supplementary Note 17)

The bird according to any one of Supplementary Notes 14 to 16,
wherein the loss-of-function pigment-expression gene is a mutant gene having a base sequence consisting of the base sequence of the wild-type pigment-expression gene with frameshift mutation, and its eye pigment expression function is suppressed.

### (Supplementary Note 18)

The bird according to any one of Supplementary Notes 14 to 17,
wherein the loss-of-function pigment-expression gene is a mutant gene having a base sequence consisting of the base sequence of the wild-type eye pigment-expression gene with splice mutation, and its eye pigment expression function is suppressed.

### (Supplementary Note 19)

The bird according to any one of Supplementary Notes 14 to 18,
wherein the pigment-expression gene is an SLC45A2 gene.

### (Supplementary Note 20)

The bird according to any one of Supplementary Notes 14 to 17,
wherein the eye pigment-expression gene is an SLC45A2 gene, and
a loss-of-function SLC45A2 gene is a mutant gene of a wild-type SLC45A2 gene having mutation in a second exon, and its eye pigment expression function is suppressed.

### (Supplementary Note 21)

The bird according to Supplementary Note 20,
wherein a base sequence of SEQ ID NO: 3 in the second exon of the wild-type SLC45A2 gene is deleted in the loss-of-function SLC45A2 gene.

### (Supplementary Note 22)

The bird according to any one of Supplementary Notes 19 to 21,
wherein the SLC45A2 gene is a gene that includes a polynucleotide (S) below:
(S) any of polynucleotides (S1) to (S7) below:
(S1) a polynucleotide having a base sequence of SEQ ID NO: 1;
(S2) a polynucleotide that encodes a protein having an eye pigment expression function and that has a base sequence consisting of the base sequence of (S1) above with deletion, substitution, insertion, and/or addition of several bases;
(S3) a polynucleotide that encodes a protein having an eye pigment expression function and that has a base sequence having 80% or more identity to the base sequence of (S1) above;
(S4) a polynucleotide that encodes a protein having an eye pigment expression function and that has a base sequence complementary to a polynucleotide capable of hybridizing to the polynucleotide having the base sequence of (S1) above under a stringent condition;
(S5) a polynucleotide encoding a protein having an amino acid sequence of SEQ ID NO: 2;
(S6) a polynucleotide encoding a protein that has an eye pigment expression function and that has an amino acid sequence consisting of the amino acid sequence of SEQ ID NO: 2 with deletion, substitution, insertion, and/or addition of several amino acids; and
(S7) a polynucleotide encoding a protein that has an eye pigment expression function and that has an amino acid sequence having 80% or more identity to the amino acid sequence of SEQ ID NO: 2.

### (Supplementary Note 23)

The bird according to any one of Supplementary Notes 19 to 22,
wherein the loss-of-function pigment-expression gene is a loss-of-function SLC45A2 gene, and
the loss-of-function SLC45A2 gene is a gene that includes a polynucleotide (M) below:
   (M) any of polynucleotides (M1) to (M7) below:
   (M1) a polynucleotide having a base sequence of SEQ ID NO: 1;
   (M2) a polynucleotide that encodes a protein whose eye pigment expression function is suppressed and that has a base sequence consisting of the base sequence of (M1) above with deletion, substitution, insertion, and/or addition of several bases;
   (M3) a polynucleotide that encodes a protein whose eye pigment expression function is suppressed and that has a base sequence having 80% or more identity to the base sequence of (M1) above;
   (M4) a polynucleotide that encodes a protein whose eye pigment expression function is suppressed and that has a base sequence complementary to a polynucleotide capable of hybridizing to the polynucleotide having the base sequence of (M1) above under a stringent condition;
   (M5) a polynucleotide encoding a protein having an amino acid sequence of SEQ ID NO: 2;
   (M6) a polynucleotide encoding a protein whose eye pigment expression function is suppressed and that has an amino acid sequence consisting of the amino acid sequence of SEQ ID NO: 2 with deletion, substitution, insertion, and/or addition of several amino acids; and
   (M7) a polynucleotide encoding a protein whose eye pigment expression function is suppressed and that has an amino acid sequence having 80% or more identity to the amino acid sequence of SEQ ID NO: 2.

### (Supplementary Note 24)

The bird according to any one of Supplementary Notes 19 to 23,
wherein the loss-of-function pigment-expression gene is a loss-of-function SLC45A2 gene, and
the loss-of-function SLC45A2 gene is a gene that includes a polynucleotide (T) below:
   (T) any of polynucleotides (T1) to (T4) below:
   (T1) a polynucleotide having a base sequence consisting of the base sequence of SEQ ID NO: 1 with deletion of the bases from position 546 to position 615;
   (T2) a polynucleotide that encodes a protein whose eye pigment expression function is suppressed and that has a base sequence consisting of the base sequence of the polynucleotide of (T1) above with deletion, substitution, insertion, and/or addition of several bases;
   (T3) a polynucleotide that encodes a protein whose eye pigment expression function is suppressed and that has a base sequence having 80% or more identity to the base sequence of the polynucleotide of (T1) above; and
   (T4) a polynucleotide that encodes a protein whose eye pigment expression function is suppressed and that has a base sequence complementary to a polynucleotide capable of hybridizing to the polynucleotide having the base sequence of (T1) above under a stringent condition.

### (Supplementary Note 25)

The bird according to any one of Supplementary Notes 14 to 24,
wherein the bird is selected from the group consisting of a chicken, a quail, a turkey, a domestic duck, a goose, an ostrich, a dove, a guinea fowl, a pheasant, a duck, and an emu.

### (Supplementary Note 26)

The bird according to any one of Supplementary Notes 14 to 25,
wherein the bird is male, and
the bird is homozygous for the loss-of-function pigment-expression gene.

### <Portion of Bird>

### (Supplementary Note 27)

A portion of the bird according to any one of Supplementary Notes 14 to 26.

### (Supplementary Note 28)

The portion according to Supplementary Note 27
wherein the portion is an edible portion.

### <Production Method>

### (Supplementary Note 29)

A bird production method including a step of mating a male bird with a female bird,
wherein the male bird is the bird according to Supplementary Note 26, and
the female bird has a wild-type eye pigment-expression gene on a Z chromosome.

### (Supplementary Note 30)

The production method according to Supplementary Note 29, including a step of selecting a bird in which a function of the eye pigment-expression gene is lost from birds obtained through the mating or progenies thereof.

### (Supplementary Note 31)

The production method according to Supplementary Note 29 or 30, including a step of selecting the bird according to Supplementary Note 26 from target male birds prior to the mating.

### (Supplementary Note 32)

The production method according to Supplementary Note 29 or 30, including a step of breeding the bird according to Supplementary Note 26 from target male birds prior to the mating.

### (Supplementary Note 33)

The production method according to Supplementary Note 31 or 32,
wherein the loss-of-function pigment-expression gene is a mutant gene having the base sequence consisting of the base sequence of a wild-type eye pigment-expression gene with deletion, substitution, insertion, and/or addition of one or several bases, and its eye pigment expression function is suppressed.

### (Supplementary Note 34)

The production method according to Supplementary Note 33,
wherein the loss-of-function pigment-expression gene is a mutant gene having a base sequence consisting of the base sequence of the wild-type eye pigment-expression gene with deletion of at least some bases, and its eye pigment expression function is suppressed.

### (Supplementary Note 35)

The production method according to any one of Supplementary Notes 31 to 34,
wherein the loss-of-function pigment-expression gene is a mutant gene having a base sequence consisting of the base sequence of the wild-type eye pigment-expression gene with frameshift mutation, and its eye pigment expression function is suppressed.

### (Supplementary Note 36)

The production method according to any one of Supplementary Notes 31 to 35,
wherein the loss-of-function pigment-expression gene is a mutant gene having a base sequence consisting of the base sequence of the wild-type eye pigment-expression gene with splice mutation, and its eye pigment expression function is suppressed.

### (Supplementary Note 37)

The production method according to any one of Supplementary Notes 31 to 36,
wherein the eye pigment-expression gene is an SLC45A2 gene.

### (Supplementary Note 38)

The production method according to any one of Supplementary Notes 31 to 37,
wherein the eye pigment-expression gene is an SLC45A2 gene, and
a loss-of-function SLC45A2 gene is a mutant gene of a wild-type SLC45A2 gene having mutation in a second exon, and its eye pigment expression function is suppressed.

### (Supplementary Note 39)

The production method according to Supplementary Note 38,
wherein a base sequence of SEQ ID NO: 3 in the second exon of the wild-type SLC45A2 gene is deleted in the loss-of-function SLC45A2 gene.

### (Supplementary Note 40)

The production method according to any one of Supplementary Notes 37 to 39,
wherein the SLC45A2 gene is a gene that includes a polynucleotide (S) below:
(S) any of polynucleotides (S1) to (S7) below:
(S1) a polynucleotide having a base sequence of SEQ ID NO: 1;
(S2) a polynucleotide that encodes a protein for expressing an eye pigment and that has a base sequence consisting of the base sequence of (S1) above with deletion, substitution, insertion, and/or addition of several bases;
(S3) a polynucleotide that encodes a protein for expressing an eye pigment and that has a base sequence having 80% or more identity to the base sequence of (S1) above;
(S4) a polynucleotide that encodes a protein for expressing an eye pigment and that has a base sequence complementary to a polynucleotide capable of hybridizing to the polynucleotide having the base sequence of (S1) above under a stringent condition;
(S5) a polynucleotide encoding a protein having an amino acid sequence of SEQ ID NO: 2;
(S6) a polynucleotide encoding a protein for expressing an eye pigment and that has an amino acid sequence consisting of the amino acid sequence of SEQ ID NO: 2 with deletion, substitution, insertion, and/or addition of several amino acids; and
(S7) a polynucleotide encoding a protein for expressing an eye pigment and that has an amino acid sequence having 80% or more identity to the amino acid sequence of SEQ ID NO: 2.

### (Supplementary Note 41)

The production method according to any one of Supplementary Notes 37 to 40,
wherein the loss-of-function pigment-expression gene is a loss-of-function SLC45A2 gene, and
the loss-of-function SLC45A2 gene is a gene that includes a polynucleotide (M) below:
   (M) any of polynucleotides (M1) to (M7) below:
   (M1) a polynucleotide having a base sequence of SEQ ID NO: 1;
   (M2) a polynucleotide that encodes a protein whose eye pigment expression function is suppressed and that has a base sequence consisting of the base sequence of (M1) above with deletion, substitution, insertion, and/or addition of several bases;
   (M3) a polynucleotide that encodes a protein whose eye pigment expression function is suppressed and that has a base sequence having 80% or more identity to the base sequence of (M1) above;
   (M4) a polynucleotide that encodes a protein whose eye pigment expression function is suppressed and that has a base sequence complementary to a polynucleotide capable of hybridizing to the polynucleotide having the base sequence of (M1) above under a stringent condition;
   (M5) a polynucleotide encoding a protein having an amino acid sequence of SEQ ID NO: 2;
   (M6) a polynucleotide encoding a protein whose eye pigment expression function is suppressed and that has an amino acid sequence consisting of the amino acid sequence of SEQ ID NO: 2 with deletion, substitution, insertion, and/or addition of several amino acids; and
   (M7) a polynucleotide encoding a protein whose eye pigment expression function is suppressed and that has an amino acid sequence having 80% or more identity to the amino acid sequence of SEQ ID NO: 2.

### (Supplementary Note 42)

The production method according to any one of Supplementary Notes 37 to 41,
wherein the loss-of-function pigment-expression gene is a loss-of-function SLC45A2 gene, and
the loss-of-function SLC45A2 gene is a gene that includes a polynucleotide (T) below:
   (T) any of polynucleotides (T1) to (T4) below:
   (T1) a polynucleotide having a base sequence consisting of the base sequence of SEQ ID NO: 1 with deletion of the bases from position 545 to position 615;
   (T2) a polynucleotide that encodes a protein whose eye pigment expression function is suppressed and that has a base sequence consisting of the base sequence of the polynucleotide of (T1) above with deletion, substitution, insertion, and/or addition of several bases;
   (T3) a polynucleotide that encodes a protein whose eye pigment expression function is suppressed and that has a base sequence having 80% or more identity to the base sequence of the polynucleotide of (T1) above; and
   (T4) a polynucleotide that encodes a protein whose eye pigment expression function is suppressed and that has a base sequence complementary to a polynucleotide capable of hybridizing to the polynucleotide having the base sequence of (T1) above under a stringent condition.

### (Supplementary Note 43)

The production method according to any one of Supplementary Notes 29 to 42,
wherein the bird is selected from the group consisting of a chicken, a quail, a turkey, a domestic duck, a goose, an ostrich, a dove, a guinea fowl, a pheasant, a duck, and an emu.

### <Bird Production Method (Second Production Method)>

### (Supplementary Note 44)

A bird production method including a step of mating a male bird with a female bird,
wherein the male bird and/or the female bird is the bird according to any one of Supplementary Notes 14 to 26.

### (Supplementary Note 45)

The bird production method according to Supplementary Note 44, including a step of selecting a male bird and/or a female bird from progenies obtained through the mating,
wherein in the selection,
when the bird is a male bird, the male bird is heterozygous for the eye pigment-expression gene on the Z chromosome and has a loss-of-function pigment-expression gene and a wild-type eye pigment-expression gene, and
when the bird is a female bird, the female bird has a loss-of-function eye pigment-expression gene on the Z chromosome.

### <Bird Production Method (Third Production Method)

### (Supplementary Note 46)

A bird production method including:
a step of sexing a bird inside each egg of a bird egg population based on a color of eyes of the bird, and
a step of obtaining a progeny using the bird that has been sexed,
wherein the sexing is conducted using the bird sexing method according to any one of Supplementary Notes 1 to 13.

### (Supplementary Note 47)

The bird production method according to Supplementary Note 46, including a step of selecting a male bird and/or a female bird from progenies obtained through the mating,
wherein in the selection,
when the bird is a male bird, the male bird is heterozygous for the eye pigment-expression gene on the Z chromosome and has a loss-of-function pigment-expression gene and a wild-type eye pigment-expression gene, and
when the bird is a female bird, the female bird has a loss-of-function eye pigment-expression gene on the Z chromosome.

### <Bird Egg Population>

### (Supplementary Note 48)

A bird egg population,
wherein when a bird inside the egg is a male bird, the male bird is heterozygous for an eye pigment-expression gene on a Z chromosome and has a loss-of-function pigment-expression gene and a wild-type eye pigment-expression gene, and
when a bird inside the egg is a female bird, the female bird has a loss-of-function eye pigment-expression gene on a Z chromosome.

### (Supplementary Note 49)

The egg population according to Supplementary Note 48,
wherein the egg population is the egg population according to any one of Supplementary Notes 1 to 13.

### (Supplementary Note 50)

The bird egg population according to Supplementary Note 48 or 49 to be used in the bird sexing method according to any one of Supplementary Notes 1 to 13.

### <Sexing Apparatus>

### (Supplementary Note 51)

A sexing apparatus including:
a light source for irradiating a sexing target egg selected from a bird egg population with light;
an imaging unit for imaging the sexing target egg in a state in which a content thereof is visible due to irradiation with light from the light source and outputting image data containing an eye of a bird inside the sexing target egg; and
a sexing unit for sexing the bird inside the sexing target egg based on the image data.

### (Supplementary Note 52)

The sexing apparatus according to Supplementary Note 51, further including:
a darkroom; and
a transport unit for transporting the sexing target egg to the darkroom,
wherein the light source and the imaging unit are provided in the darkroom,
the light source irradiates the sexing target egg placed in the darkroom with light,
the imaging unit images the sexing target egg irradiated with light from the light source in the darkroom, and
the imaging unit is configured to be capable of imaging a lateral side of the sexing target egg.

### (Supplementary Note 53)

The sexing apparatus according to Supplementary Note 51 or 52,
wherein the light source is provided at a position where a straight line connecting a sharp end and a blunt end of the sexing target egg coincides with an optical axis of the light source,
the imaging unit is configured to be capable of imaging a lateral side of the sexing target egg, and
the sexing apparatus further includes a rotating unit for rotating the sexing target egg about a rotation axis extending along the straight line connecting the sharp end and the blunt end of the sexing target egg.

### (Supplementary Note 54)

The sexing apparatus according to any one of Supplementary Notes 51 to 53, further including a removing unit for removing an egg determined as a male by the sexing unit from the bird egg population.

### (Supplementary Note 55)

The sexing apparatus according to any one of Supplementary Notes 51 to 54,
wherein the bird egg population is the bird egg population according to any one of Supplementary Notes 48 to 50.

### (Supplementary Note 56)

The sexing apparatus according to any one of Supplementary Notes 51 to 55,
wherein the sexing unit detects an image of an eyeball of a bird from images based on the image data and executes predetermined image processing on the eyeball image, and
when the eyeball image after the image processing satisfies a first condition, the bird is determined as a male, and when the eyeball image after the image processing satisfies a second condition, the bird is determined as a female.

### (Supplementary Note 57)

The sexing apparatus according to any one of Supplementary Notes 51 to 55,
wherein the sexing unit sexes a bird inside the sexing target egg using a trained model that previously learned a data set containing images that includes eyes of birds and are each labeled as a male or a female.

### (Supplementary Note 58)

A sexing apparatus including:
a light source for light irradiation;
an imaging unit for imaging an imaging target and outputting image data;
a memory storing a sexing program; and
a processor,
wherein by executing the sexing program, the processor
   operates the light source and allows the light source to irradiate a sexing target egg selected from a bird egg population with light,
   operates the imaging unit and allows the imaging unit to image the sexing target egg in a state in which a content thereof is visible due to irradiation with light from the light source and output image data containing an eye of a bird inside the sexing target egg, and
   sexes the bird inside the sexing target egg based on the image data.

### (Supplementary Note 59)

The sexing apparatus according to Supplementary Note 58,
wherein the processor detects an image of an eyeball of a bird from images based on the image data and executes predetermined image processing on the eyeball image, and
when the eyeball image after the image processing satisfies a first condition, the bird is determined as a male, and when the eyeball image after the image processing satisfies a second condition, the bird is determined as a female.

### (Supplementary Note 60)

The sexing apparatus according to Supplementary Note 58,
wherein the processor sexes a bird inside the sexing target egg using a trained model that previously learned a data set containing images that includes eyes of birds and are each labeled as a male or a female.

### (Supplementary Note 61)

A computer readable non-transitory tangible storage medium storing a program for allowing a computer to execute sexing of a bird,
wherein the program is configured to allow a computer to
operate a light source and allow the light source to irradiate a sexing target egg selected from a bird egg population with light,
operate an imaging unit and allow the imaging unit to image the sexing target egg in a state in which a content thereof is visible due to irradiation with light from the light source and output image data containing an eye of a bird inside the sexing target egg, and
sex the bird inside the sexing target egg based on the image data.

### (Supplementary Note 62)

The storage medium according to Supplementary Note 61,
wherein the program detects an image of an eyeball of a bird from images based on the image data and executes predetermined image processing on the eyeball image, and
when the eyeball image after the image processing satisfies a first condition, the bird is determined as a male, and when the eyeball image after the image processing satisfies a second condition, the bird is determined as a female.

### (Supplementary Note 63)

The storage medium according to Supplementary Note 61,
wherein the program sexes a bird inside the sexing target egg using a trained model that previously learned a data set containing images that includes eyes of birds and are each labeled as a male or a female.

### Industrial Applicability

As described above, the bird of the present disclosure has the loss-of-function eye pigment-expression gene on the Z chromosome and can be sexed. Accordingly, the present disclosure is very useful, for example, in the fields of bird breeding, livestock industry, and the like.

[Sequence Listing]

## Claims

1. A bird sexing method comprising a step of sexing a bird inside each egg of a bird egg population based on a color of eyes of the bird,
wherein when the bird inside the egg is a male bird, the male bird is heterozygous for an eye pigment-expression gene on a Z chromosome and has a loss-of-function pigment-expression gene and a wild-type eye pigment-expression gene, and
when the bird inside the egg is a female bird, the female bird has a loss-of-function eye pigment-expression gene on a Z chromosome.

2. The sexing method according to claim 1, comprising a step of preparing the egg population by mating a male parent bird with a female parent bird prior to the sexing,
wherein the male parent bird has the loss-of-function eye pigment-expression gene, and is homozygous for the loss-of-function pigment-expression gene, and
the female parent bird has the wild-type eye pigment-expression gene.

3. The sexing method according to claim 1 or 2,
wherein the loss-of-function pigment-expression gene is a mutant gene having a base sequence consisting of the base sequence of the wild-type eye pigment-expression gene with deletion, substitution, insertion, and/or addition of one or several bases, and its eye pigment expression function is suppressed.

4. The sexing method according to claim 3,
wherein the loss-of-function pigment-expression gene is a mutant gene having a base sequence consisting of the base sequence of the wild-type eye pigment-expression gene with deletion of at least some bases, and its eye pigment expression function is suppressed.

5. The sexing method according to any one of claims 1 to 4,
wherein the loss-of-function pigment-expression gene is a mutant gene having a base sequence consisting of the base sequence of the wild-type eye pigment-expression gene with frameshift mutation, and its eye pigment expression function is suppressed.

6. The sexing method according to any one of claims 1 to 5,
wherein the loss-of-function pigment-expression gene is a mutant gene having a base sequence consisting of the base sequence of the wild-type eye pigment-expression gene with splice mutation, and its eye pigment expression function is suppressed.

7. The sexing method according to any one of claims 1 to 6,
wherein the eye pigment-expression gene is an SLC45A2 gene.

8. The sexing method according to any one of claims 1 to 7,
wherein the eye pigment-expression gene is an SLC45A2 gene, and
a loss-of-function SLC45A2 gene is a mutant gene of a wild-type SLC45A2 gene having mutation in a second exon, and its eye pigment expression function is suppressed.

9. The sexing method according to claim 8,
wherein a base sequence of SEQ ID NO: 3 in the second exon of the wild-type SLC45A2 gene is deleted in the loss-of-function SLC45A2 gene.

10. The sexing method according to any one of claims 7 to 9,
wherein the SLC45A2 gene is a gene that includes a polynucleotide (S) below:
(S) any of polynucleotides (S1) to (S7) below:
(S1) a polynucleotide having a base sequence of SEQ ID NO: 1;
(S2) a polynucleotide that encodes a protein having an eye pigment expression function and that has a base sequence consisting of the base sequence of (S1) above with deletion, substitution, insertion, and/or addition of several bases;
(S3) a polynucleotide that encodes a protein having an eye pigment expression function and that has a base sequence having 90% or more identity to the base sequence of (S1) above;
(S4) a polynucleotide that encodes a protein having an eye pigment expression function and that has a base sequence complementary to a polynucleotide capable of hybridizing to the polynucleotide having the base sequence of (S1) above under a stringent condition;
(S5) a polynucleotide encoding a protein having an amino acid sequence of SEQ ID NO: 2;
(S6) a polynucleotide encoding a protein that has an eye pigment expression function and that has an amino acid sequence consisting of the amino acid sequence of SEQ ID NO: 2 with deletion, substitution, insertion, and/or addition of several amino acids; and
(S7) a polynucleotide encoding a protein that has an eye pigment expression function and that has an amino acid sequence having 90% or more identity to the amino acid sequence of SEQ ID NO: 2.

11. The sexing method according to any one of claims 7 to 10,
wherein the loss-of-function pigment-expression gene is a loss-of-function SLC45A2 gene, and
the loss-of-function SLC45A2 gene is a gene that includes a polynucleotide (M) below:
(M) any of polynucleotides (M1) to (M7) below:
(M1) a polynucleotide having a base sequence of SEQ ID NO: 1;
(M2) a polynucleotide that encodes a protein whose eye pigment expression function is suppressed and that has a base sequence consisting of the base sequence of (M1) above with deletion, substitution, insertion, and/or addition of several bases;
(M3) a polynucleotide that encodes a protein whose eye pigment expression function is suppressed and that has a base sequence having 80% or more identity to the base sequence of (M1) above;
(M4) a polynucleotide that encodes a protein whose eye pigment expression function is suppressed and that has a base sequence complementary to a polynucleotide capable of hybridizing to the polynucleotide having the base sequence of (M1) above under a stringent condition;
(M5) a polynucleotide encoding a protein having an amino acid sequence of SEQ ID NO: 2;
(M6) a polynucleotide encoding a protein whose eye pigment expression function is suppressed and that has an amino acid sequence consisting of the amino acid sequence of SEQ ID NO: 2 with deletion, substitution, insertion, and/or addition of several amino acids; and
(M7) a polynucleotide encoding a protein whose eye pigment expression function is suppressed and that has an amino acid sequence having 80% or more identity to the amino acid sequence of SEQ ID NO: 2.

12. The sexing method according to any one of claims 7 to 11,
wherein the loss-of-function pigment-expression gene is a loss-of-function SLC45A2 gene, and
the loss-of-function SLC45A2 gene is a gene that includes a polynucleotide (T) below:
(T) any of polynucleotides (T1) to (T4) below:
(T1) a polynucleotide having a base sequence consisting of the base sequence of SEQ ID NO: 1 with deletion of the bases from position 545 to position 615;
(T2) a polynucleotide that encodes a protein whose eye pigment expression function is suppressed and that has a base sequence consisting of the base sequence of the polynucleotide of (T1) above with deletion, substitution, insertion, and/or addition of several bases;
(T3) a polynucleotide that encodes a protein whose eye pigment expression function is suppressed and that has a base sequence having 80% or more identity to the base sequence of the polynucleotide of (T1) above; and
(T4) a polynucleotide that encodes a protein whose eye pigment expression function is suppressed and that has a base sequence complementary to a polynucleotide capable of hybridizing to the polynucleotide having the base sequence of (T1) above under a stringent condition.

13. The sexing method according to any one of claims 1 to 12,
wherein the bird is selected from the group consisting of a chicken, a quail, a turkey, a domestic duck, a goose, an ostrich, a dove, a guinea fowl, a pheasant, a duck, and an emu.

14. A bird having a loss-of-function eye pigment-expression gene on a Z chromosome.

15. The bird according to claim 14,
wherein the loss-of-function pigment-expression gene is a mutant gene having a base sequence consisting of a base sequence of a wild-type eye pigment-expression gene with deletion, substitution, insertion, and/or addition of one or several bases, and its eye pigment expression function is suppressed.

16. The bird according to claim 15,
wherein the loss-of-function pigment-expression gene is a mutant gene having a base sequence consisting of the base sequence of the wild-type pigment-expression gene with deletion of at least some bases, and its eye pigment expression function is suppressed.

17. The bird according to any one of claims 14 to 16,
wherein the loss-of-function pigment-expression gene is a mutant gene having a base sequence consisting of the base sequence of the wild-type pigment-expression gene with frameshift mutation, and its eye pigment expression function is suppressed.

18. The bird according to any one of claims 14 to 17,
wherein the loss-of-function pigment-expression gene is a mutant gene having a base sequence consisting of the base sequence of the wild-type eye pigment-expression gene with splice mutation, and its eye pigment expression function is suppressed.

19. The bird according to any one of claims 14 to 18,
wherein the pigment-expression gene is an SLC45A2 gene.

20. The bird according to any one of claims 14 to 17,
wherein the eye pigment-expression gene is an SLC45A2 gene, and
a loss-of-function SLC45A2 gene is a mutant gene of a wild-type SLC45A2 gene having mutation in a second exon, and its eye pigment expression function is suppressed.

21. The bird according to claim 20,
wherein a base sequence of SEQ ID NO: 3 in the second exon of the wild-type SLC45A2 gene is deleted in the loss-of-function SLC45A2 gene.

22. The bird according to any one of claims 19 to 21,
wherein the SLC45A2 gene is a gene that includes a polynucleotide (S) below:
(S) any of polynucleotides (S1) to (S7) below:
(S1) a polynucleotide having a base sequence of SEQ ID NO: 1;
(S2) a polynucleotide that encodes a protein having an eye pigment expression function and that has a base sequence consisting of the base sequence of (S1) above with deletion, substitution, insertion, and/or addition of several bases;
(S3) a polynucleotide that encodes a protein having an eye pigment expression function and that has a base sequence having 80% or more identity to the base sequence of (S1) above;
(S4) a polynucleotide that encodes a protein having an eye pigment expression function and that has a base sequence complementary to a polynucleotide capable of hybridizing to the polynucleotide having the base sequence of (S1) above under a stringent condition;
(S5) a polynucleotide encoding a protein having an amino acid sequence of SEQ ID NO: 2;
(S6) a polynucleotide encoding a protein that has an eye pigment expression function and that has an amino acid sequence consisting of the amino acid sequence of SEQ ID NO: 2 with deletion, substitution, insertion, and/or addition of several amino acids; and
(S7) a polynucleotide encoding a protein that has an eye pigment expression function and that has an amino acid sequence having 80% or more identity to the amino acid sequence of SEQ ID NO: 2.

23. The bird according to any one of claims 19 to 22,
wherein the loss-of-function pigment-expression gene is a loss-of-function SLC45A2 gene, and
the loss-of-function SLC45A2 gene is a gene that includes a polynucleotide (M) below:
(M) any of polynucleotides (M1) to (M7) below:
(M1) a polynucleotide having a base sequence of SEQ ID NO: 1;
(M2) a polynucleotide that encodes a protein whose eye pigment expression function is suppressed and that has a base sequence consisting of the base sequence of (M1) above with deletion, substitution, insertion, and/or addition of several bases;
(M3) a polynucleotide that encodes a protein whose eye pigment expression function is suppressed and that has a base sequence having 80% or more identity to the base sequence of (M1) above;
(M4) a polynucleotide that encodes a protein whose eye pigment expression function is suppressed and that has a base sequence complementary to a polynucleotide capable of hybridizing to the polynucleotide having the base sequence of (M1) above under a stringent condition;
(M5) a polynucleotide encoding a protein having an amino acid sequence of SEQ ID NO: 2;
(M6) a polynucleotide encoding a protein whose eye pigment expression function is suppressed and that has an amino acid sequence consisting of the amino acid sequence of SEQ ID NO: 2 with deletion, substitution, insertion, and/or addition of several amino acids; and
(M7) a polynucleotide encoding a protein whose eye pigment expression function is suppressed and that has an amino acid sequence having 80% or more identity to the amino acid sequence of SEQ ID NO: 2.

24. The bird according to any one of claims 19 to 23,
wherein the loss-of-function pigment-expression gene is a loss-of-function SLC45A2 gene, and
the loss-of-function SLC45A2 gene is a gene that includes a polynucleotide (T) below:
(T) any of polynucleotides (T1) to (T4) below:
(T1) a polynucleotide having a base sequence consisting of the base sequence of SEQ ID NO: 1 with deletion of the bases from position 546 to position 615;
(T2) a polynucleotide that encodes a protein whose eye pigment expression function is suppressed and that has a base sequence consisting of the base sequence of the polynucleotide of (T1) above with deletion, substitution, insertion, and/or addition of several bases;
(T3) a polynucleotide that encodes a protein whose eye pigment expression function is suppressed and that has a base sequence having 80% or more identity to the base sequence of the polynucleotide of (T1) above; and
(T4) a polynucleotide that encodes a protein whose eye pigment expression function is suppressed and that has a base sequence complementary to a polynucleotide capable of hybridizing to the polynucleotide having the base sequence of (T1) above under a stringent condition.

25. The bird according to any one of claims 14 to 24,
wherein the bird is selected from the group consisting of a chicken, a quail, a turkey, a domestic duck, a goose, an ostrich, a dove, a guinea fowl, a pheasant, a duck, and an emu.

26. The bird according to any one of claims 14 to 25,
wherein the bird is male, and
the bird is homozygous for the loss-of-function pigment-expression gene.

27. A portion of the bird according to any one of claims 14 to 26.

28. The portion according to claim 27
wherein the portion is an edible portion.

29. A bird production method comprising a step of mating a male bird with a female bird,
wherein the male bird is the bird according to claim 26, and
the female bird has a wild-type eye pigment-expression gene on a Z chromosome.

30. The production method according to claim 29, comprising a step of selecting a bird in which a function of the eye pigment-expression gene is lost from birds obtained through the mating or progenies thereof.

31. The production method according to claim 29 or 30, comprising a step of selecting the bird according to claim 26 from target male birds prior to the mating.

32. The production method according to claim 29 or 30, comprising a step of breeding the bird according to claim 26 from target male birds prior to the mating.

33. The production method according to claim 31 or 32,
wherein the loss-of-function pigment-expression gene is a mutant gene having a base sequence consisting of the base sequence of the wild-type eye pigment-expression gene with deletion, substitution, insertion, and/or addition of one or several bases, and its eye pigment expression function is suppressed.

34. The production method according to claim 33,
wherein the loss-of-function pigment-expression gene is a mutant gene having a base sequence consisting of the base sequence of the wild-type eye pigment-expression gene with deletion of at least some bases, and its eye pigment expression function is suppressed.

35. The production method according to any one of claims 31 to 34,
wherein the loss-of-function pigment-expression gene is a mutant gene having a base sequence consisting of the base sequence of the wild-type eye pigment-expression gene with frameshift mutation, and its eye pigment expression function is suppressed.

36. The production method according to any one of claims 31 to 35,
wherein the loss-of-function pigment-expression gene is a mutant gene having a base sequence consisting of the base sequence of the wild-type eye pigment-expression gene with splice mutation, and its eye pigment expression function is suppressed.

37. The production method according to any one of claims 31 to 36,
wherein the eye pigment-expression gene is an SLC45A2 gene.

38. The production method according to any one of claims 31 to 37,
wherein the eye pigment-expression gene is an SLC45A2 gene, and
a loss-of-function SLC45A2 gene is a mutant gene of a wild-type SLC45A2 gene having mutation in a second exon, and its eye pigment expression function is suppressed.

39. The production method according to claim 38,
wherein a base sequence of SEQ ID NO: 3 in the second exon of the wild-type SLC45A2 gene is deleted in the loss-of-function SLC45A2 gene.

40. The production method according to any one of claims 37 to 39,
wherein the SLC45A2 gene is a gene that includes a polynucleotide (S) below:
(S) any of polynucleotides (S1) to (S7) below:
(S1) a polynucleotide having a base sequence of SEQ ID NO: 1;
(S2) a polynucleotide that encodes a protein for expressing an eye pigment and that has a base sequence consisting of the base sequence of (S1) above with deletion, substitution, insertion, and/or addition of several bases;
(S3) a polynucleotide that encodes a protein for expressing an eye pigment and that has a base sequence having 80% or more identity to the base sequence of (S1) above;
(S4) a polynucleotide that encodes a protein for expressing an eye pigment and that has a base sequence complementary to a polynucleotide capable of hybridizing to the polynucleotide having the base sequence of (S1) above under a stringent condition;
(S5) a polynucleotide encoding a protein having an amino acid sequence of SEQ ID NO: 2;
(S6) a polynucleotide encoding a protein for expressing an eye pigment and that has an amino acid sequence consisting of the amino acid sequence of SEQ ID NO: 2 with deletion, substitution, insertion, and/or addition of several amino acids; and
(S7) a polynucleotide encoding a protein for expressing an eye pigment and that has an amino acid sequence having 80% or more identity to the amino acid sequence of SEQ ID NO: 2.

41. The production method according to any one of claims 37 to 40,
wherein the loss-of-function pigment-expression gene is a loss-of-function SLC45A2 gene, and
the loss-of-function SLC45A2 gene is a gene that includes a polynucleotide (M) below:
(M) any of polynucleotides (M1) to (M7) below:
(M1) a polynucleotide having a base sequence of SEQ ID NO: 1;
(M2) a polynucleotide that encodes a protein whose eye pigment expression function is suppressed and that has a base sequence consisting of the base sequence of (M1) above with deletion, substitution, insertion, and/or addition of several bases;
(M3) a polynucleotide that encodes a protein whose eye pigment expression function is suppressed and that has a base sequence having 80% or more identity to the base sequence of (M1) above;
(M4) a polynucleotide that encodes a protein whose eye pigment expression function is suppressed and that has a base sequence complementary to a polynucleotide capable of hybridizing to the polynucleotide having the base sequence of (M1) above under a stringent condition;
(M5) a polynucleotide encoding a protein having an amino acid sequence of SEQ ID NO: 2;
(M6) a polynucleotide encoding a protein whose eye pigment expression function is suppressed and that has an amino acid sequence consisting of the amino acid sequence of SEQ ID NO: 2 with deletion, substitution, insertion, and/or addition of several amino acids; and
(M7) a polynucleotide encoding a protein whose eye pigment expression function is suppressed and that has an amino acid sequence having 80% or more identity to the amino acid sequence of SEQ ID NO: 2.

42. The production method according to any one of claims 37 to 41,
wherein the loss-of-function pigment-expression gene is a loss-of-function SLC45A2 gene, and
the loss-of-function SLC45A2 gene is a gene that includes a polynucleotide (T) below:
(T) any of polynucleotides (Tl) to (T4) below:
(T1) a polynucleotide having a base sequence consisting of the base sequence of SEQ ID NO: 1 with deletion of the bases from position 545 to position 615;
(T2) a polynucleotide that encodes a protein whose eye pigment expression function is suppressed and that has a base sequence consisting of the base sequence of the polynucleotide of (T1) above with deletion, substitution, insertion, and/or addition of several bases;
(T3) a polynucleotide that encodes a protein whose eye pigment expression function is suppressed and that has a base sequence having 80% or more identity to the base sequence of the polynucleotide of (T1) above; and
(T4) a polynucleotide that encodes a protein whose eye pigment expression function is suppressed and that has a base sequence complementary to a polynucleotide capable of hybridizing to the polynucleotide having the base sequence of (T1) above under a stringent condition.

43. The production method according to any one of claims 29 to 42,
wherein the bird is selected from the group consisting of a chicken, a quail, a turkey, a domestic duck, a goose, an ostrich, a dove, a guinea fowl, a pheasant, a duck, and an emu.

44. A bird production method comprising a step of mating a male bird with a female bird,
wherein the male bird and/or the female bird is the bird according to any one of claims 14 to 26.

45. The bird production method according to claim 44, comprising a step of selecting a male bird and/or a female bird from progenies obtained through the mating,
wherein in the selection,
when the bird is a male bird, the male bird is heterozygous for the eye pigment-expression gene on the Z chromosome and has a loss-of-function pigment-expression gene and a wild-type eye pigment-expression gene, and
when the bird is a female bird, the female bird has a loss-of-function eye pigment-expression gene on the Z chromosome.

46. A bird production method comprising:
a step of sexing a bird inside each egg of a bird egg population based on a color of eyes of the bird, and
a step of obtaining a progeny using the bird that has been sexed,
wherein the sexing is conducted using the bird sexing method according to any one of claims 1 to 13.

47. The bird production method according to claim 46, comprising a step of selecting a male bird and/or a female bird from progenies obtained through the mating,
wherein in the selection,
when the bird is a male bird, the male bird is heterozygous for the eye pigment-expression gene on the Z chromosome and has a loss-of-function pigment-expression gene and a wild-type eye pigment-expression gene, and
when the bird is a female bird, the female bird has a loss-of-function eye pigment-expression gene on the Z chromosome.

48. A bird egg population,
wherein when a bird inside the egg is a male bird, the male bird is heterozygous for an eye pigment-expression gene on a Z chromosome and has a loss-of-function pigment-expression gene and a wild-type eye pigment-expression gene, and
when a bird inside the egg is a female bird, the female bird has a loss-of-function eye pigment-expression gene on a Z chromosome.

49. The egg population according to claim 48,
wherein the egg population is the egg population according to any one of claims 1 to 13.

50. The bird egg population according to claim 48 or 49 to be used in the bird sexing method according to any one of claims 1 to 13.

51. A sexing apparatus comprising:
a light source for irradiating a sexing target egg selected from a bird egg population with light;
an imaging unit for imaging the sexing target egg in a state in which a content thereof is visible due to irradiation with light from the light source and outputting image data containing an eye of a bird inside the sexing target egg; and
a sexing unit for sexing the bird inside the sexing target egg based on the image data.

52. The sexing apparatus according to claim 51, further comprising:
a darkroom; and
a transport unit for transporting the sexing target egg to the darkroom,
wherein the light source and the imaging unit are provided in the darkroom,
the light source irradiates the sexing target egg placed in the darkroom with light,
the imaging unit images the sexing target egg irradiated with light from the light source in the darkroom, and
the imaging unit is configured to be capable of imaging a lateral side of the sexing target egg.

53. The sexing apparatus according to claim 51 or 52,
wherein the light source is provided at a position where a straight line connecting a sharp end and a blunt end of the sexing target egg coincides with an optical axis of the light source,
the imaging unit is configured to be capable of imaging a lateral side of the sexing target egg, and
the sexing apparatus further includes a rotating unit for rotating the sexing target egg about a rotation axis extending along the straight line connecting the sharp end and the blunt end of the sexing target egg.

54. The sexing apparatus according to any one of claims 51 to 53, further comprising a removing unit for removing an egg determined as a male by the sexing unit from the bird egg population.

55. The sexing apparatus according to any one of claims 51 to 54,
wherein the bird egg population is the bird egg population according to any one of claims 48 to 50.

56. The sexing apparatus according to any one of claims 51 to 55,
wherein the sexing unit detects an image of an eyeball of a bird from images based on the image data and executes predetermined image processing on the eyeball image, and
when the eyeball image after the image processing satisfies a first condition, the bird is determined as a male, and when the eyeball image after the image processing satisfies a second condition, the bird is determined as a female.

57. The sexing apparatus according to any one of claims 51 to 55,
wherein the sexing unit sexes a bird inside the sexing target egg using a trained model that previously learned a data set containing images that includes eyes of birds and are each labeled as a male or a female.
